# EUROPÄISCHE PATENTSCHRIFT

(11) **EP 1 893 327 B1**
(45) Veröffentlichungstag und Bekanntmachung des Hinweises auf die Patenterteilung: **21.04.2010**
(21) Anmeldenummer: 06763722.3
(22) Anmeldetag: 14.06.2006
(51) Int. Cl.: B01J 2/12, B01J 8/10, A61K 31/519

(54) **EINRICHTUNG ZUR FÜHRUNG EINES GASES FÜR VORRICHTUNGEN ZUM BEHANDELN KÖRNIGEN GUTES DURCH TROCKNEN, FILMCOATEN ODER BESCHICHTEN, INSBESONDERE ZULUFTEINHEIT UND VORRICHTUNG MIT EINER DERARTIGEN EINRICHTUNG**
INSTALLATION FOR GUIDING A GAS FOR DEVICES USED TO TREAT GRANULAR PRODUCTS BY DRYING, FILM-COATING OR COATING, ESPECIALLY AN INCOMING AIR UNIT, AND DEVICE COMPRISING ONE SUCH INSTALLATION
INSTALLATION POUR GUIDER UN GAZ DESTINE A DES DISPOSITIFS SERVANT A TRAITER UNE MATIERE GRANULEUSE PAR SECHAGE, APPLICATION D'UN FILM OU ENDUCTION, NOTAMMENT UNITE D'ALIMENTATION ET DISPOSITIF COMPORTANT UNE TELLE INSTALLATION

(30) Priorität: 17.06.2005 DE 102005028168
(43) Veröffentlichungstag der Anmeldung: 05.03.2008
(73) Patentinhaber: Boehringer Ingelheim Pharma GmbH & Co. KG, 55216 Ingelheim am Rhein (DE)
(72) Erfinder: MAIER, Johann-Georg, 72511 Bingen (DE); HILLER, Dietmar, 88457 Kirchdorf (DE); SAUTER, Frank, 89613 Oberstadion-Hundersingen (DE); JÄGER, Eberhard, 88453 Erolzheim (DE); BECKER, Daniel, 88433 Assmannshardt (DE); MISSEL, Roland, 88400 Biberach (DE)
(74) Vertreter: Hammann, Heinz
(86) Internationale Anmeldenummer: PCT/EP2006/063227
(87) Internationale Veröffentlichungsnummer: WO 2006/134133

(56) Entgegenhaltungen:
- WO-A-2004/093881
- US-A- 4 563 315
- US-B1- 6 569 462

## Beschreibung

Die Erfindung betrifft eine Einrichtung zur Führung eines Gases für Vorrichtungen zum Behandeln körnigen Gutes durch Trocknen, Filmcoaten oder Beschichten, insbesondere Zulufteinheit, im Einzelnen mit den Merkmalen aus dem Oberbegriff von Anspruch 1, ferner eine Vorrichtung zum Behandeln körnigen Gutes durch Trocknen, Filmcoaten oder Beschichten mit einer derartigen Einrichtung, die Verwendung der Vorrichtung zur Herstellung von Dabigatran etexilate Pellets oder Dipyridamol Retardformen sowie die unter Verwendung der Vorrichtung hergestellten Produkte.

Vorrichtungen zum Behandeln körnigen Gutes durch Trocknen, Filmcoaten oder Beschichten sind in einer Vielzahl von Ausführungen bekannt. Stellvertretend wird auf die nachfolgend genannten Druckschriften verwiesen:
EP 0 080 199 A2
EP 0 732 882 B1
WO 01/26601
US 4,563,315

Die Druckschrift EP 0 080 199 A2 offenbart eine Vorrichtung zum Behandeln körnigen Gutes durch Trocknen, Filmcoaten oder Beschichten. Diese umfasst eine um eine waagerechte Achse drehbar gelagerte Trommel, in welcher Ein- und Auslassleitungen für ein Gas zum Trocknen eines in der Trommel enthaltenen Gutes an einen Tauchkörper angeschlossen sind, der Ein- und Austrittsöffnungen für das Gas aufweist und innerhalb der Trommel eine Stellung einnimmt, in der er in das Gut eintaucht. Der Tauchkörper ist als ein vom Gut durchströmbarer Tunnel ausgeführt, in dessen Inneren zumindest ein Teil der Ein- und Austrittsöffnungen für das Gas angeordnet sind. Der Tunnel ist in Einbaulage in Strömungsrichtung des Gutes ausgerichtet. Befindet sich Gut in der Trommel wird beispielsweise aus einer Düse austretendes Befeuchtungsmedium von unten und von oben her auf das Gut gesprüht, während die Trommel rotiert. Dabei strömt die obere Schicht des Gutes unter einem Winkel zur Waagerechten abwärts. In diese abwärts strömende Schicht des Gutes taucht der Tauchkörper ein. Dieser umfasst einen Zwischenboden, der im Zusammenwirken mit axialen und in Strömungsrichtung vorgesehenen Trennwänden diesen in eine ringförmige Einlasskammer für Trocknungsgas und eine flache Auslasskammer unterteilt. Die Einlasskammer ist an eine Einlassleitung angeschlossen und die Auslasskammer an eine Auslassleitung. Der Zwischenboden weist schlitzförmige Eintrittsöffnungen auf, die sich über die gesamte Breite erstrecken und dem Trocknungsgas den Weg aus der Einlasskammer in den Innenraum des vom Tauchkörper gebildeten Tunnels freigeben. Bei dieser Lösung ist die Menge des der direkten Trocknung unterzogenen Gutes abhängig von der Tunnelgeometrie, der Eintauchtiefe und der Anordnung der Austrittskanäle und damit jeweils konkret an den Einzelfall anzupassen. Dabei ist zur optimalen Trocknung zum einen eine lichte Weite des Eintrittsquerschnittes in Abhängigkeit der Breite der Trommel zu gewährleisten und ferner ein bestimmter vordefinierter Befüllungsgrad der Trommel, der in Einbaulage des Tauchkörpers immer eine Befüllung dessen zwischen Zwischenboden und Zwischendecke gewährleistet. Die Trocknung des Gutes erfolgt von unten, was bei großem Befüllungsgrad und entsprechend großer Tunnelhöhe zu unbefriedigenden und ungleichmäßigen Trocknungsergebnissen führen kann. Zwar wird hier aufgrund der schlitzförmigen Ausführung der Auslassöffnungen für das Trocknungsgas eine direkte Trocknung über einen größeren Bereich in Strömungsrichtung gewährleistet, allerdings erfolgt die Versorgung des gesamten Ringraumes nur über eine einzige Einlasskammer. Nachteilig ist ferner die komplexe Geometrie des den Tunnel bildenden und die Auslassöffnungen für das Trocknungsgas tragenden Elements, welche eine konkrete Anpassung an die Auslegung der Trommel erfordert, die nicht gegebene Austauschbarkeit und fehlende Anpassbarkeit an veränderliche Füllstände.

Aus der Druckschrift EP 0 732 882 B1 ist eine Ausführung einer Vorrichtung zum Behandeln körnigen Gutes vorbekannt, welche eine Trocknung von körnigen Gut in einer drehbar gelagerten Beschichtungspfanne über mit perforierten Hohlkörpern versehene Luftauslassmittel ermöglicht, die in das Gut eintauchbar sind. Die Auslassöffnungen sind hier an rohrförmigen Elementen, die sich radial von einem Verteilerkanal erstrecken und damit in einer festen Position gegenüber dem Verteilerkanal angeordnet. Je nach Befüllungsgrad der Trommel sind dabei unterschiedliche Eintauchtiefen der Luftauslassmittel in das Gut gegeben, was sich in unterschiedlichen Trocknungsergebnissen wiederspiegelt. Ferner erstrecken sich aufgrund der radial ausgerichteten Anordnung der Auslassöffnungen und deren Querschnitte die Wirkungs- und damit Trocknungsbereiche jeweils nur ringförmig um diese.

Zur Lösung der Problematik sind aus der WO 01/26601 teleskopförmige Hohlkörper vorbekannt, die längenverstellbar sind, sich vertikal vom Verteilerkanal erstrecken und in die bewegte Masse des zu behandelnden Gutes eintauchbar sind. Die Hohlkörper weisen in ihrem distalen Teil einen länglichen Querschnitt mit zugespitzten Enden auf, der am Boden unter Bildung der Auslassöffnungen offen ist und ferner an den Seitenwänden perforierte Bereiche zum Trocknungsgasaustritt aufweist. Der wirksame Trocknungsbereich kann somit vergrößert werden. Nachteilig ist jedoch auch hier der stark eingegrenzte Trocknungsbereich um die Auslassöffnungen an den teleskopförmigen Hohlkörpern, der sich um den einzelnen Hohlkörper erstreckt, wobei die Trocknungswirkung in radialer Richtung abnimmt.

Eine gattungsgemäße Vorrichtung zum Behandeln körnigen Gutes ist aus der Druckschrift US 4,563,315 vorbekannt. Diese weist eine Luftzuführeinheit auf, umfassend eine zentrale und einen Verteilerkanal beschreibende Versorgungsleitung, die sich innerhalb einer rotierbaren Trommel erstreckt und welche mit in radialer Richtung ausgerichteten und in Funktionskanäle mündenden Verbindungskanälen verbunden ist. Die einzelnen Verbindungskanäle erstrecken sich ausgehend vom Verteilerkanal in Richtung des Innenumfanges der Trommel. Von den Verbindungkanälen erstrecken sich die Auslassöffnungen tragenden Funktionskanäle in Form von rohrförmigen Gebilden in Längsrichtung in der Trommel. Dabei sind eine Mehrzahl derartiger Einheiten aus Verbindungs- und Funktionskanälen vorgesehen, die über einen Teilbereich in Umfangsrichtung innerhalb der Trommel und in dieser Richtung zueinander beabstandet angeordnet sind. Diese sind ortsfest gegenüber dem zentralen Versorgungsbeziehungsweise Verteilerkanal angeordnet.

Der Erfindung liegt daher die Aufgabe zugrunde, eine Einrichtung zur Führung eines Gases in Vorrichtungen zum Behandeln körnigen Gutes derart weiterzuentwickeln, dass die genannten Nachteile vermieden werden und mit konstruktiv einfachen Mitteln ein großflächiger und für einen Großteil des zu behandelnden Gutes leicht zugänglicher Trocknungsbereich bereitgestellt wird, wobei die Trocknungsintensität unabhängig vom Befüllungsgrad derartiger Vorrichtungen möglichst gleich sein sollte. Ferner sollte die erfindungsgemäße Lösung sich durch einen einfachen Aufbau auszeichnen und auch zum Nachrüsten in bestehenden Vorrichtungen geeignet sein.

Die erfindungsgemäße Lösung ist durch die Merkmale der Ansprüche 1 und 2 charakterisiert. Eine Vorrichtung zum Behandeln körnigen Gutes mit einer erfindungsgemäßen Einrichtung zur Führung eines Gases ist durch die Merkmale von Anspruch 33 charakterisiert. Vorteilhafte Ausgestaltungen sind jeweils in den Unteransprüchen beschrieben.

Eine Einrichtung zur Führung eines Gases für Vorrichtungen zum Behandeln körnigen Gutes durch Trocknen, Filmcoaten oder Beschichten, insbesondere eine Zulufteinheit, umfassend einen zentralen Verteilerkanal, der mit einer Gasbereitstellungseinrichtung verbindbar ist und mit von diesem beabstandet angeordneten und in axialer Richtung zueinander beabstandet angeordneten Auslässen für das Gas über Verbindungskanäle verbunden ist, weist erfindungsgemäß einen eine Vielzahl von Auslässen aufweisenden und zum Verteilerkanal beabstandet angeordneten Funktionskanal auf. Der Funktionskanal umfasst gemäß einem ersten Lösungsansatz wenigstens zwei zueinander axial beabstandet angeordnete Einlassbereiche, wobei jeder der Einlassbereiche mit dem Verteilerkanal über einen Verbindungskanal verbunden ist. Die Auslässe sind in axialer Richtung am Funktionskanal betrachtet zwischen der Mündung zweier in axialer Richtung benachbart angeordneter Verbindungskanäle in den Funktionskanal angeordnet.

Der Begriff Kanal ist funktional als strömungsführende Einheit zu verstehen, wobei keinerlei Beschränkungen bezüglich der konstruktiven Ausführung und Geometrie bestehen. Der einzelne Kanal kann dabei von einem Hohlkörper gebildet werden, in ein Element eingearbeitet oder von Leitungselementen gebildet sein.

Gemäß einem zweiten Lösungsansatz ist der Funktionskanal über nur einen Verbindungskanal mit dem Verteilerkanal verbunden, wobei der Funktionskanal in einem Winkel zum Verbindungskanal angeordnet ist und sich von diesem wegerstreckt. Vorzugsweise ist der Funktionskanal fliegend am Verbindungskanal gelagert.

Mit der erfindungsgemäßen Lösung wird somit eine Führung der Trocknungsluft gewährleistet, die durch einen großen Wirkbereich charakterisiert ist und ferner durch die Umlenkung im gewünschten Winkel gegenüber dem zu trocknenden Gut austritt.

Die erfindungsgemäße Ausführung ermöglicht beim Einsatz in Vorrichtungen zum Behandeln körnigen Gutes durch Trocknen, Filmcoaten oder Beschichten, insbesondere pharmazeutischer Partikel in Form von Pellets oder Granulaten gemäß Anspruch 31 einen großflächigen Bereich über die Vorrichtungsbreite und in Strömungsrichtung mit gleichmäßiger Trocknungsintensität für das in der Vorrichtung, insbesondere der drehbaren Trommel enthaltene und bei Rotation über den Funktionskanal bewegte Gut. Das Gut wird dabei bei Rotation der Trommel in einer Fließbewegung gehalten und je nach Anordnung eine bestimmte Schichtdicke über den Funktionskanal geführt, wobei während der Abwärtsbewegung des Gutes dieses gleichmäßig dem aus den Auslässen austretenden Trocknungsgas ausgesetzt wird. Das über den Funktionskanal geführte Gut wird somit direkt dem Gas ausgesetzt, d.h. getrocknet. Eine vorherige Führung des Gutes, wie bei der Tunnellösung ist nicht erforderlich. Die erfindungsgemäße Einrichtung kann dabei in Vorrichtungen zum Behandeln körnigen Gutes zum Einsatz gelangen, welche reine Trocknungsfunktion und Trocknungsfunktion nach Beschichtungen, Wirkstoffsprühen oder Filmcoaten übernehmen. Dazu sind der Einrichtung zum Führen eines Gases Beschichtungs- und/oder Befeuchtungseinrichtungen in Strömungsrichtung des sich entgegen der Rotationsrichtung abwärtsströmenden Gutes vorgeschaltet. Die Nachordnung der erfindungsgemäßen Einrichtung bietet den Vorteil, dass das Gut nach dem Besprühen in einer Quasi-Wirbelschicht, unter welcher eine gerichtete und geordnete Strömung verstanden wird, bei welcher eine Volumenzunahme erfolgt, indem der mittlere Teilchenabstand und damit die dem Trocknungsgas aussetzbare Oberfläche vergrößert wird, getrocknet wird, wodurch die Trocknungsperformance verbessert und die Prozesszeiten verringert werden.

Die erfindungsgemäße Lösung ermöglicht neben einer Erhöhung der Trocknungsintensität auch eine Verringerung der Prozesszeiten durch kürzere Aufheizzeiten sowie die Möglichkeit der Erhöhung der Sprührate ohne Erhöhung der Zuluftmenge oder der Änderung zusätzlicher Parameter. Aufgrund des großflächigen Austrittes des Trocknungsgases ergeben sich weniger Verwirbelungen in der Trommel als bei den bekannten Lösungen.

Die erfindungsgemäß gebildete Trocknungszone weist dabei beispielsweise eine Länge parallel zur Rotationsachse des Kessels auf, welche dem 0, 1 bis 0,9 fachen, vorzugsweise 0,1 bis 0, 5 fachen Betrag der Breite der Trommel, inbesondere im Bereich der Rotationsachse oder des Funktionskanals entspricht.

Die Auslässe sind erfindungsgemäß am Außenumfang des Funktionskanals vorgesehen, wobei diese wenigstens an der zum Verteilerkanal gerichteten Seite bzw. Oberfläche angeordnet sind und somit beim Einsatz in einer Vorrichtung zum Behandeln körnigen Gutes gegenüber diesem derart positioniert sind, dass ein Gleiten einer oberen Schicht des abwärts strömenden Gutes darüber gewährleistet ist.

Gemäß einer Ausführungsform sind die Auslässe auch an dem vom Verteilerkanal weggerichteten Bereich der Außenfläche des Funktionskanals angeordnet. Dies bietet den Vorteil, dass der Funktionskanal beliebig tief in das Gut eintauchbar ist und aufgrund des Austritts von Trocknungsgas in entgegengesetzter Richtung der Trocknungseffekt auch bei unterschiedlichen Befüllungsgraden gewährleistet wird, wobei hier auch eine Verwirbelung in unteren, d.h. im Bereich der Trommelwandung liegenden Schichtbereichen gewährleistet wird. Eine derartige Ausführung ist vor allem bei in seiner Lage gegenüber dem Verteilerkanal nicht veränderbaren Funktionskanal von Vorteil.

Die Auslässe überdecken jeweils wenigstens einen vordefinierten Flächenbereich am Außenumfang des Funktionskanals. Zur Vergrößerung des wirksamen Auslassquerschnittes sind die einzelnen Auslässe durch einen Auslassquerschnitt im Bereich von 0,1 bis 1 mm, bevorzugt 0,2 bis 0,6 mm, insbesondere etwa 0,5 mm charakterisiert und mit einem vordefinierten Abstand im Bereich von 0,5 bis 2 mm, bevorzugt 0,8 bis 1,5 mm, insbesondere etwa 1 mm zueinander in axialer Richtung und senkrecht dazu zueinander angeordnet. Vorzugsweise werden diese von engmaschig perforierten Flächenbereichen gebildet, d.h. große Häufigkeit der Anordnung der Auslässe und mit geringem Querschnitt. Die Querschnittsgeometrie kann kreisförmig, vieleckig oder schlitzförmig, beispielsweise auch wabenförmig sein. Jede andere Geometrie ist ebenfalls denkbar. Die Auslassquerschnittsgröße ist dabei auch abhängig von der Größe des zu trocknenden Gutes.

Bezüglich der Lagezuordnung zwischen wenigstens einem Teilbereich des Verteilerkanals, vorzugsweise des gesamten Verteilerkanals und dem Austrittsöffnungen tragenden Funktionskanal werden folgende Möglichkeiten unterschieden:
a) parallel
b) in einem Winkel
zueinander.

Vorzugsweise wird die parallele Lage gewählt, da diese beim Einsatz in Vorrichtungen zur Behandlung körniger Güter eine gleichmäßige Beaufschlagung des Gutes in Strömungsrichtung und quer dazu über den Funktionskanal mit Trocknungsluft ermöglicht. Ferner ist diese Ausführung damit auch in bereits bestehende Vorrichtungen gut integrierbar, d.h. es ist auch eine leichte Nachrüstbarkeit, beispielsweise durch Austausch gegeben. Die Winkellage ist nur bei einer entsprechenden Einbausituation für den Verteilerkanal interessant, die trotz nichtparalleler Anordnung des Verteilerkanals eine axiale Erstreckung des Funktionskanals parallel zur Rotationsachse der Beschichtungstrommel ermöglicht. Ferner ermöglicht diese Einbaulage es, eine optimale Luftführung in der Quasi-Wirbelschicht einzustellen und mit einer Mindestfüllung zu arbeiten.

Vorzugsweise erfolgt die Anordnung von Verteilerkanal und den Verbindungskanälen senkrecht zueinander. Dies gilt in Analogie auch für den Funktionskanal und die Verbindungskanäle. Die Anordnung aller in einer gemeinsamen Ebene stellt eine konstruktiv besonders einfach zu realisierende Ausführung dar.

Bezüglich der gewählten Querschnittsgeometrien bestehen für die Ausführung der einzelnen Kanäle keine Beschränkungen. Die Auslegung erfolgt dabei in Abhängigkeit der gewählten Zuluftmenge und gewünschten Strömungsverhältnisse. Der Funktionskanal ist zur Erzielung einer Verringerung des Strömungswiderstandes im Querschnitt betrachtet vorzugsweise flügelartig ausgeführt, d.h. in Strömungsrichtung des Gutes betrachtet nach vorn hin geneigter. Der Querschnitt wird von durch abgerundete Übergänge miteinander verbundenen Ober- und Unterseiten gebildet, wobei die Oberseite und die Unterseite im Querschnitt betrachtet jeweils durch die Aneinanderreihung unterschiedlich ausgestalteter und ausgerichteter Flächenbereiche charakterisiert ist, die zum Zweck der besseren Führung des Gutes jeweils in Strömungsrichtung zu den Endbereichen hin geneigt sind.

Bezüglich der Lagezuordnung zwischen Funktions- und Verteilerkanal werden erfindungsgemäß Ausführungen mit der Möglichkeit einer Lageveränderung eingesetzt. Diese bieten die Möglichkeit der optimalen Anpassung an unterschiedliche Befüllgrade, wobei diese entweder zwischen unterschiedlichen Prozessen oder während eines Prozesses realisiert werden können.

Lageveränderungen werden durch
a) Verdreh- oder Verschwenkbarkeit des Funktionskanals, beispielsweise Verdrehbarkeit um seine Lagerachse und/oder
b) Verschiebbarkeit senkrecht zum Verteilerkanal, d.h. auf diesen zu oder von diesem weg
erzeugt.

Die Verdrehbarkeit wird im einfachsten Fall über formschlüssige oder kraftschlüssige Verbindungsmittel zwischen Funktionskanal und Verbindungskanal realisiert, wobei jeweils Mittel zur Feststellung vorgesehen sind. Die Verdrehbarkeit kann stufenlos oder in Stufen erfolgen. Im letztgenannten Fall umfassen die Verbindungsmittel vorzugsweise eine Vielzahl von in Umfangsrichtung der Drehachse angeordneten Rastmitteln, welche vorzugsweise gleich ausgeführt sind und mit einer bestimmten Teilung, d.h. Abstand zueinander in Umfangsrichtung angeordnet sind, welcher den Verdrehwinkel beschreibt.

Im Fall b) erfolgt die Verschiebbarkeit entlang der Längsachsen der Verbindungskanäle. Die konstruktive Umsetzung kann unterschiedlich erfolgen. Dabei werden Lösungen mit
1) Verbindungskanälen mit konstanter Länge und
2) Verbindungskanälen mit einstellbarer Länge
unterschieden.

Gemäß 1) ist im einfachsten Fall der Funktionskanal dazu an seinen Endbereichen in einem Schieber gelagert, der wiederum in einer Führung am den Verbindungskanal bildenden Element entlang der Längsachse dessen bzw. parallel dazu geführt ist. Vorzugsweise wird eine Gleitführung, beispielsweise aus Teflon verwendet. Dabei ist der Schieber vorzugsweise derart ausgeführt, dass dieser eine größere Fläche in Längsrichtung als die Führung überdeckt und somit in jeder Funktionsstellung nur ein Übertritt zwischen Verbindungskanal und den Einlässen am Funktionskanal in den einander gegenüberstehenden Bereichen am Verbindungskanal und dem Funktionskanal freigegeben ist. Vorzugsweise sind die Austritte aus dem Verbindungskanal im Führungsbereich durch eine perforierte Fläche am den Verbindungskanal bildenden Element charakterisiert. Andere Ausführungen sind denkbar.

Gemäß 2) kann auf die Gleitführung verzichtet werden. Der einzelne Verbindungskanal ist teleskopförmig ausgeführt. Durch Veränderung der Länge wird der Funktionskanal näher an den Verteilerkanal oder weiter weg von diesem bewegt.

In beiden Fällen ist zur Realisierung der Lageänderung wenigstens eine Stelleinrichtung vorgesehen. Diese kann in der Einrichtung integriert oder zumindest teilweise außerhalb angeordnet sein. Bei dieser kann es sich um
- mechanische
- hydraulische
- pneumatische
- elektronische
- eine Kombination aus vorgenannten

Lösungen handeln. Die Betätigung dieser kann manuell, servounterstützt oder automatisch erfolgen. Dazu ist eine Vorwahleinrichtung vorgesehen, an der ein Sollwert für eine einen Verstellweg wenigstens mittelbar charakterisierende Größe vorgebbar ist, und die mit einer Übertragungseinheit verbunden ist, die die Sollgröße in eine Stellgröße umsetzt. Gemäß einer besonders einfach zu realisierenden Lösung umfasst die Übertragungseinheit ein Getriebe, welches eine Drehbewegung an der als Vorwahleinrichtung fungierenden Betätigungseinrichtung in eine Längsbewegung umwandelt, beispielsweise in Form einer Zahnrad-/Zahhstangeneinheit oder eines Winkeltriebs mit Innengewinde am Abtriebsrad, das mit einem Außengewinde einer im Abtriebsrad geführten Stange zusammenwirkt, wobei die Stange mit dem Schieber verbunden ist.

Durch die beschriebenen Möglichkeiten der Lageveränderung kann die Einrichtung mit unterschiedlichen Befüllgraden betrieben werden, ohne das Trocknungsergebnis zu beeinträchtigen.

Die Möglichkeit der Verdrehung und der Verschiebbarkeit des Funktionskanals kann mit separaten Stelleinrichtungen oder aber einer, beide Funktionen getrennt voneinander oder zwangsgekoppelt ausführenden Stelleinrichtung umgesetzt werden.

Die Materialwahl für die einzelnen Elemente der Einrichtung erfolgt in Abhängigkeit der Anforderungen an das zu behandelnde Gut. Vorzugsweise werden durch Umformen oder Fügen aus Blechhalbzeugen hergestellte Blechformteile verwendet. Diese bestehen aufgrund der vorherrschenden physikalischen und chemischen Beanspruchungen vorzugsweise aus Edelstahl. Der Verbindungskanal wird dabei im einfachsten Fall von einem einen Hohlkörper bildenden Element, welches jeweils mit dem Verteilerkanal und dem Verbindungskanal über Öffnungsbereiche in strömungsführender Verbindung steht, und sich vom Verteilerkanal wegerstreckt, gebildet. Auch der Verteilerkanal und der Funktionskanal werden vorzugsweise von einem Hohlkörper gebildet.

Vorzugsweise wird der den Funktionskanal bildende Hohlkörper wenigstens teilweise von einem Lochblech, vorzugsweise mit Stützgerüst oder einer perforierten Metallfolie gebildet. Vorzugsweise findet inertes Material Verwendung.

Gemäß einer weiteren Ausführungsform werden die Auslässe von einem Lochblech mit Stützgerüst oder einer perforierten Metallfolie gebildet, die einen Teilbereich des Außenumfanges des den Funktionskanal beschreibenden Hohlkörpers bilden. Diese sind vorzugsweise lösbar und damit austauschbar mit dem Hohlkörper verbunden, wobei diese großflächige Öffnungsbereiche am Außenumfang des Hohlkörpers überdecken. Dies ermöglicht eine einfache Herausnahme zu Reinigungszwecken oder eine Änderung der Durchtrittsquerschnitte.

Gemäß einer Weiterentwicklung ist auch der Funktionskanal teleskopartig ausgeführt. Dies ermöglicht die Bereitstellung unterschiedlicher Wirkungsweiten zur Anpassung an unterschiedliche Breiten von Beschichtungseinrichtungen. Ferner ist dadurch auch eine einfache Anpassung an unterschiedliche Auslassweiten am Verteilerkanal gegeben, insbesondere wenn die erfindungsgemäße Einrichtung unter Ausnutzung bestehender Verteilerkanäle in bestehenden Anlagen nachgerüstet werden soll.

Bei den zu behandelnden Gütern handelt es sich um körnige Partikel, Granulate oder Pellets, vorzugsweise um pharmazeutische Produkte, beispielsweise um die in der WO 03/074056 beschriebenen Dabigatran etexilate (3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester) Pellets, oder um die in EP 0032562 beschriebenen Dipyridamol Retardformen, die Bestandteil der Präparate Asasantin^{®} und Aggrenox^{®} darstellen.

Die vorliegende Erfindung bezieht sich auch auf die Verwendung der erfindungsgemäßen Vorrichtung zur Dabigatran etexilate Pellets und Dipyridamol Retardformen sowie auf diese unter Verwendung der erfindungsgemäßen Vorrichtung hergestellten Produkte.

Als Trocknungsgas findet vorzugsweise Luft Verwendung.

Die erfindungsgemäße Lösung wird nachfolgend anhand von Figuren erläutert. Darin ist im einzelnen folgendes dargestellt:
- Figuren 1a bis 1d: verdeutlichen in schematisch vereinfachter Darstellung theoretisch mögliche Grundausführungen erfindungsgemäß ausgeführter Einrichtungen;
- Figuren 2a bis 2e: verdeutlicht eine Ausführung einer Einrichtung gemäß Figur 1c in mehreren Ansichten;
- Figur 2f: verdeutlicht in schematisiert vereinfachter Darstellung den Aufbau einer mechanischen Lösung zur Verschiebung des Funktionskanals;
- Figuren 3a und 3b: verdeutlichen in schematisch vereinfachter Darstellung den Grundaufbau und das Funktionsprinzip einer erfindungsgemäß gestalteten Vorrichtung zum Behandeln körnigen Gutes;
- Figur 4a, 4b und 4c: verdeutlichen mögliche Anordnungen der Auslassöffnungen am Funktionskanal und
- Figur 5: verdeutlicht in stark schematisierter Darstellung den Grundaufbau einer Einrichtung gemäß dem zweiten Lösungsansatz.

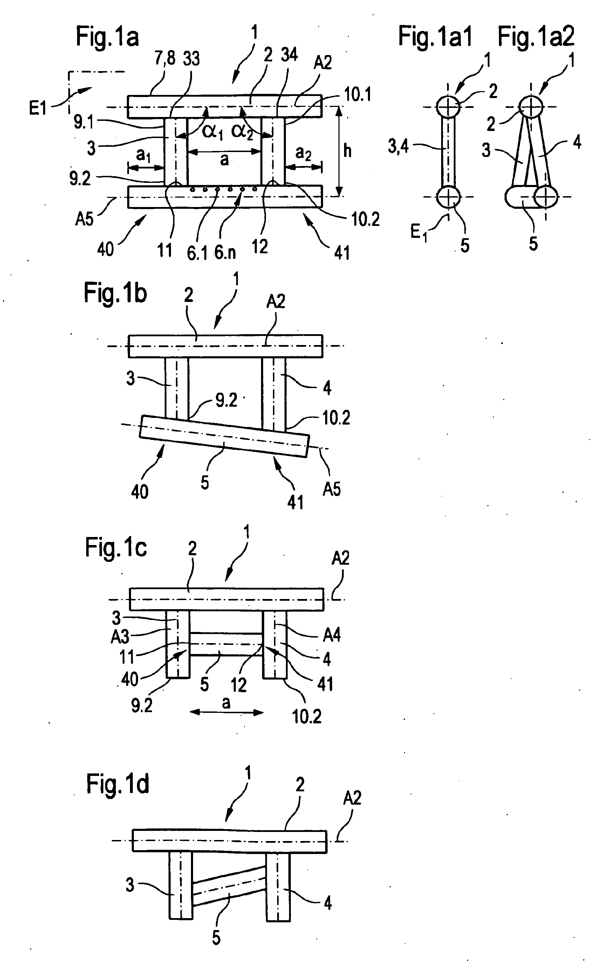

Die Figuren 1a bis 1d verdeutlichen in schematisch vereinfachter Darstellung den Grundaufbau einer erfindungsgemäß ausgeführten Einrichtung 1 für den Einsatz in Vorrichtungen zur Behandlung körnigen Gutes durch Trocknen, Filmcoaten oder Beschichten, insbesondere für Vorrichtungen zur Behandlung pharmazeutischer Partikel. Diese umfasst einen zentralen Verteilkanal 2, der mit einer hier nicht dargestellten Zuluftversorgungseinrichtung beim Einsatz in den vorgenannten Vorrichtungen verbindbar ist und von welchem sich wenigsten zwei in axialer Richtung betrachtet beabstandet zueinander angeordnete Verbindungskanäle 3 und 4 in einem Winkel α1 und α2 erstrecken. Es ist ein Zusammenführungskanal, welcher als eigentlicher Funktionskanal 5 in Form des Auslasskanals fungiert vorgesehen, der beabstandet zum Verteilerkanal 2 angeordnet ist und mit den zwei in axialer Richtung zueinander beabstandet und einander benachbart angeordneten Verbindungskanälen 3 und 4 verbunden ist und diese somit miteinander verbindet. Dieser umfasst wenigstens zwei Einlassbereiche 11 und 12 und eine Vielzahl von Austrittsöffnungen 6.1 bis 6.n für die über die Kanäle 3 und 4 in diesen mündende Zuluft. Entsprechend der Anordnung und der Kopplung der einzelnen Kanäle miteinander können mehrere Grundvarianten unterschieden werden. Alle Varianten können dabei mit fester Zuordnung hinsichtlich der Lage zwischen dem Verteilerkanal 2 und dem Funktionskanal 5 oder aber veränderbarer Zuordnung ausgeführt werden. Zuordnungsänderungen sind im wesentlichen durch die mögliche Verdrehbarkeit des Funktionskanals 5 um seine Achse A5, insbesondere Lagerachse und/oder die Verschiebbarkeit gegenüber dem Verteilerkanal 2 unter Veränderung des Abstandes h in Vertikalrichtung zum Verteilerkanal 2 charakterisiert. Bei allen Ausführungen bestehen für die gewählten Querschnittsgeometrien der einzelnen Kanäle 3, 4 sowie des Verteilerkanals 2 und des Funktionskanals 5 keine Beschränkungen. Die Figuren 1a bis 1c verdeutlichen dabei die möglichen Zuordnungen und Anbindungen nur grundsätzlich und schematisiert. Auf die Darstellung der die einzelnen Kanäle - Verteilerkanal 2, Verbindungskanal 3, 4 und Funktionskanal 5 - bildenden oder diese tragenden Elemente in ihrer konstruktiven Ausgestaltung wurde verzichtet. Ferner sind die wiedergegebenen Querschnitte der einzelnen Kanäle nur beispielhaft. Der Verteilerkanal 2 ist auf seiner von der Anschlussseite zur Zuluftversorgungseinrichtung weggewandten Stirnseite 20 geschlossen.

Figur 1a verdeutlich eine erste Ausführung mit parallel zum Verteilerkanal 2 angeordnetem und Austrittsöffnungen 6.1 bis 6.n tragenden Funktionskanal 5 in zwei Ansichten. Der Verteilerkanal 2 wird dabei von einem Höhlkörper 7, im einfachsten Fall einem rohrförmigen Element 8 beliebiger Querschnittsausführung gebildet. Dieser verläuft vorzugsweise in axialer Richtung und weist wenigstens zwei in axialer Richtung zueinander beabstandet angeordnete Austritte 33, 34 für die Zuluft auf. Die Austritte 33 und 34 liegen dabei als Öffnung oder perforierter Bereich vor. Der Funktionskanal 5 ist in einem Abstand h zum Verteilerkanal 2 angeordnet. Der Abstand h entspricht in der dargestellten Position dem Abstand in vertikaler Richtung, in Einbaulage der Einrichtung 1 dem Abstand in radialer Richtung. Die Verbindung der Austritte 33 und 34 mit dem Funktionskanal 5 erfolgt über so genannte Verbindungskanäle 3, 4. Diese erstrecken sich vom Verteilerkanal 2 weg, wobei beide einander benachbart und beabstandet in axialer Richtung zueinander angeordnet sind. Die Erstreckung der Verbindungskanäle 3 und 4 erfolgt in einem Winkel α1 und α2, vorzugsweise jeweils im gleichen Winkel, vorzugsweise einem Winkel von 90° zu einer den Verlauf des Verteilerkanals 2 in axialer Richtung beschreibenden Längsachse A2. Beide Verbindungskanäle 3 und 4 sind vorzugsweise parallel zueinander angeordnet. Diese sind jeweils mit ihrem ersten Endbereich 9.1 für den Kanal 3 und 10.1 für den Kanal 4 mit dem Verteilerkanal 2 verbunden, während der zweite Endbereich 9.2 bzw. 10.2 mit dem Funktionskanal 5 verbunden ist. Dabei mündet in der in Figur 1a dargestellten Ausführung gemäß Figur 1a1 der einzelne Verbindungskanal 3 bzw. 4 jeweils senkrecht in den Verteilerkanal 2 als auch senkrecht in den Funktionskanal 5. Alle Kanäle 2, 3, 4 und 5 sind in einer Ebene E1 angeordnet. Eine Mündung im Winkel ist entsprechend Figur 1a2 beispielhaft ebenfalls möglich.

Der Funktionskanal 5 erstreckt sich in axialer Richtung betrachtet wenigstens über den Abstand a zwischen den beiden Verbindungskanälen 3 und 4. Im dargestellten Fall erstreckt sich der Funktionskanal 5 jeweils über eine Erstreckung a1 und a2 in axialer Richtung über den Abstand a zwischen beiden Kanälen 3 und 4 und die Kanalbreiten der Verbindungskanäle 3 und 4 hinaus. Die Abstände a1 und a2 können theoretisch aber auch 0 betragen oder derart bemessen werden, dass der Funktionskanal 5 in axialer Richtung bündig mit den Verbindungskanälen 3 und 4 abschließt. Der Funktionskanal 5 ist in diesem Fall wenigstens durch das Vorsehen von Austrittsöffnungen 6.1 bis 6.n zwischen den beiden Verbindungskanälen 3 und 4 charakterisiert, vorzugsweise auch im Bereich der Erstreckungen a1 und a2.

Beide Verbindungskanäle 3 und 4 bzw. die diese charakterisierenden Längsachsen A3 und A4 sowie die Achse A2 sind vorzugsweise in einer Ebene E1 angeordnet. Dies bedeutet, dass die Kanäle 3 und 4 in Richtung senkrecht zur Axialrichtung frei von einem Versatz zueinander angeordnet sind, wie in der Ansicht von Rechts in Figur 1a1 wiedergegeben. Denkbar ist jedoch auch ein Versatz bei Anordnung des Funktionskanals 5 zwar in einer parallelen Ebene zum Verteilerkanal 2, jedoch gegenüber diesem bei theoretischer Projizierung in eine gemeinsame Ebene in einem Winkel zueinander und damit unterschiedlicher Ausrichtung der Verbindungskanäle 3 und 4.

Demgegenüber verdeutlicht Figur 1b eine Ausführung gemäß Figur 1a mit geneigter Anordnung zwischen Verteilerkanal 2 und Funktionskanal 5, welche durch die winklige Lage der Längsachsen A2 von Verteilerkanal 2 und A5 von Funktionskanal 5 zueinander wiedergegeben ist. Die Anbindung erfolgt auch hier in den Endbereichen 9.2 und 10.2 an die Verbindungskanäle 3 und 4. Vorzugsweise erfolgt auch hier eine Anordnung der Verbindungskanäle 3 und 4 in Richtung senkrecht zur Axialrichtung frei von Versatz zueinander, d.h. in einer Ebene.

Bei beiden Ausführungen gemäß der Figuren 1a und 1b kann der Funktionskanal 5 mit seinen Einlassbereichen 11 und 12 im Endbereich 40, 41 mit den Verbindungskanälen 3 und 4 verbunden sein oder aber die Kanäle 3 und 4 münden an beliebiger Stelle zwischen den beiden, dann verschlossenen Endbereichen 40, 41 in diesen. Eine Lageänderung zwischen Verteilerkanal 2 und Funktionskanal 5 ist bei den in den Figuren 1a und 1b dargestellten Ausführungen in erster Linie nur bei Teleskopierbarkeit der die Verbindungskanäle 3 und 4 bildenden Elemente und damit der Verbindungskanäle 3 und 4 gegeben.

Figur 1c verdeutlicht eine besonders vorteilhafte Ausgestaltung einer Einrichtung 1, welche durch einen konstruktiv einfachen Aufbau sowie einfach zu realisierende Möglichkeiten der Lageänderungen des Funktionskanals 5 gegenüber dem Verteilerkanal 2 charakterisiert ist. Dabei erstreckt sich der Verteilerkanal 2 entlang der Längsachse A2 und die beiden Verbindungskanäle 3 und 4 jeweils unter Bildung eines Abstandes a in axialer Richtung beabstandet zueinander und parallel. Die den Verteilerkanal 2 charakterisierende Längsachse A2 sowie die den Verlauf der Verbindungskanäle 3 und 4 beschreibenden Längsachsen A3, A4 sind vorzugsweise in einer Ebene E1 angeordnet. D.h. die Anordnung der Verbindungskanäle 3 und 4 erfolgt im gleichen Winkel α1 und α2 gegenüber der Längsachse A2 des Verteilerkanals 2. Gegenüber den Ausführungen der Figuren 1a und 1b erfolgt jedoch die Anbindung des Funktionskanals 5 nicht am Endbereich 9.2 bzw. 10.2 der Verbindungskanäle 3 und 4 sondern die Verbindungskanäle 3 und 4 sind als Blindkanäle ausgeführt, d.h. an den Endbereichen 9.2 und 10.2 jeweils verschlossen. Die Anbindung des Funktionskanals 5 erfolgt im Bereich der Erstreckung der Verbindungskanäle 3 und 4 senkrecht zur Axialrichtung, d.h. zwischen den Verbindungskanälen 3 und 4. Die axiale Erstreckung des Funktionskanals 5 entspricht dabei im wesentlichen dem axialen Abstand a der beiden Kanäle 3 und 4 zueinander. Bei dieser Ausführung mündet der Funktionskanal 5 direkt mit seinen Einlassbereichen 11 und 12, welche an den Enden 40 und 41 angeordnet sind, in die Verbindungskanäle 3 und 4. Möglichkeiten der Lageänderung von Funktionskanal 5 gegenüber dem Verteilerkanal 2 bestehen in einer Änderung des Abstandes und/oder Verdrehbarkeit des Funktionskanals 5 und damit der Auslassöffnungen 6.1 bis 6.n.

Die Figur 1d verdeutlicht eine Ausführung gemäß Figur 1c mit geneigter Anordnung des Funktionskanals 5 gegenüber dem Verteilerkanal 2.

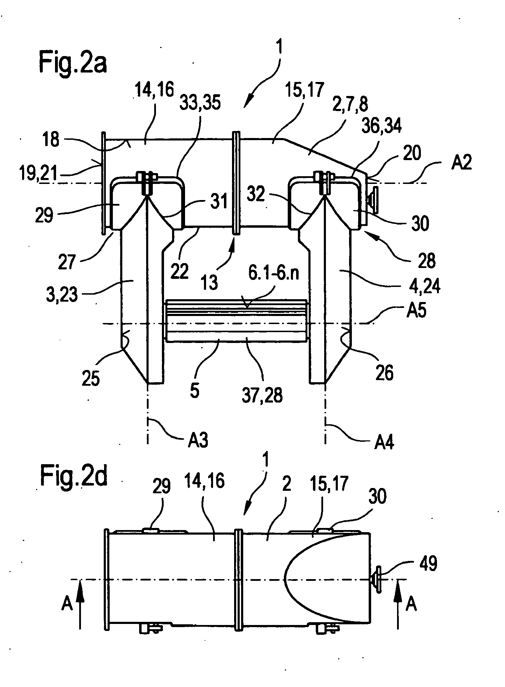

Figuren 2a bis 2e verdeutlichen in schematisch vereinfachter Darstellung eine konstruktive Ausgestaltung einer erfindungsgemäß ausgeführten Einrichtung 1 gemäß Figur 1c in mehreren Ansichten. Diese umfasst eine Verteilerkanal 2, der von einem rohrförmigen Element 8 mit vorzugsweise im wesentlichen über seine axiale Erstreckung kreisförmigen Querschnitt gebildet wird. Andere Querschnittsgeometrien sind denkbar. Jede ist jedoch durch wenigstens eine Längsachse A2, die den Verlauf des Verteilerkanals 2 in axialer Richtung beschreibt, charakterisiert. Der Verteilerkanal 2 ist mehrteilig aufgebaut und wird im dargestellten Fall beispielsweise von zwei miteinander zu einer baulichen Einheit in Form des rohrförmigen Elementes 8 zusammenfassbaren Teilelementen 14 und 15 in Form eines Grundrohres 16 und eines Aufsatzrohres 17 gebildet, die miteinander vorzugsweise lösbar verbindbar sind. Die Verbindung erfolgt im einfachsten Fall über Flanschkupplungen 13 und/oder einfache Verbindungselemente. Der Verteilerkanal 2 wird dann von der Innenwandung 18 des rohrförmigen Elementes 8 beschrieben. Das rohrförmige Element 8 ist an seiner Stirnseite 19, welche vom Grundrohr 16 gebildet wird, offen und an seiner Stirnseite 20 verschlossen. Die Öffnung 21 an der Stirnseite 19 ist mit weiteren Elementen einer Zulufteinrichtung koppelbar, beispielsweise weiteren rohrförmigen Elementen zur Führung der Zuluft sowie entweder direkt oder indirekt über weitere Zuluftführungseinrichtungen mit einer Zuluftbereitstellungseinrichtung. Vom Außenumfang 22 des den Verteilerkanal 2 bildenden rohrförmigen Elementes 8 erstrecken sich zwei Stutzen 23 und 24, welche als einseitig verschlossene Hohlkörper ausgeführt sind. Diese verlaufen senkrecht zum rohrförmigen Element 8 und beinhalten oder bilden die Verbindungskanäle 3 und 4, vorzugsweise werden diese durch die Geometrie des von diesen Hohlkörpern gebildeten Innenraumes 25 und 26 beschrieben. Die so gebildeten Innenräume 25 und 26, welche vorzugsweise auch die Verbindungskanäle 3 und 4 bilden oder aber beinhalten stehen in strömungsführender Verbindung mit dem Verteilerkanal 2, insbesondere münden in diesen. Dazu ist jeder Stutzen 23 und 24 an seinem mit dem Verteilerkanal 2 zu koppelnden Ende 27 und 28 mit einem an die Geometrie des Außenumfanges 22 des den Verteilerkanal 2 bildenden Elementes 8 angepassten Flansch 29, 30 ausgeführt. Die Öffnung 31 bzw. 32 der Stutzen in diesem Bereich kommuniziert dann mit dem Verteilerkanal 2 über an dem diesen beschreibenden Element 8 vorgesehenen Öffnungen 33, 34, wobei die Öffnung 33 den Mündungsbereich 35 von Verbindungskanal 3 mit dem Verteilerkanal 2 und die Öffnung 34 den Mündungsbereich 36 von Verbindungskanal 4 mit dem Verteilerkanal 2 beschreibt. Die Geometrie der Stutzen, insbesondere der Querschnitt der Stutzen 23 und 24 kann beliebig gewählt werden, entscheidend ist die Bildung eines Verbindungskanals 3 und 4. Vorzugsweise weisen beide Stutzen 23 und 24 den gleichen Aufbau und die gleiche Geometrie auf und sind zum Zweck einer Austauschbarkeit auch gegeneinander vorzugsweise symmetrisch bezüglich der den Verlauf der Verbindungskanäle 3 und 4 beschreibenden Längsachsen A3 und A4 aufgebaut. Die Verbindungskanäle 3 und 4 sind jeweils mit dem Austrittsöffnungen 6.1 bis 6.n tragenden Funktionskanal 5 verbunden. Dieser wird ebenfalls von einem Hohlkörper 37 gebildet, der auch als Schwert 38 bezeichnet wird. Dieser ist an den beiden Stutzen 23 und 24 gelagert. Vorzugsweise drehbar und in Vertikalrichtung verschiebbar. Die Lagerachse, welche die Achse A5 beschreibt verläuft dabei parallel zur Längsachse A2 des Verteilerkanals 2 und jeweils senkrecht zu den Längsachsen A3 und A4 der Verbindungskanäle 3 und 4.

Die Figur 2a verdeutlicht dabei eine Ansicht von vom. Erkennbar sind die parallele Anordnung der Stutzen 23 und 24 zueinander, die parallele Anordnung des rohrförmigen Elementes 8 und des den Funktionskanal 5 bildenden Hohlkörpers 37, sowie der Aufbau der Stutzen 23 und 24 und die senkrechte Anordnung zum rohrförmigen Element 8 sowie dem Hohlkörper 37. Ersichtlich sind ferner die zweiteilige Ausführung des rohrförmigen Elementes 8 sowie die Ausführung der Verbindungen zwischen den Einzelelementen des rohrförmigen Elements sowie des rohrförmigen Elementes 8 und der Stutzen 23, 24.

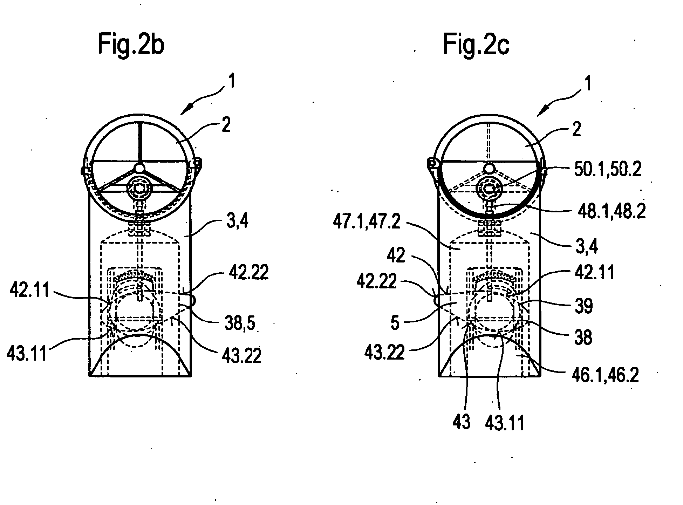

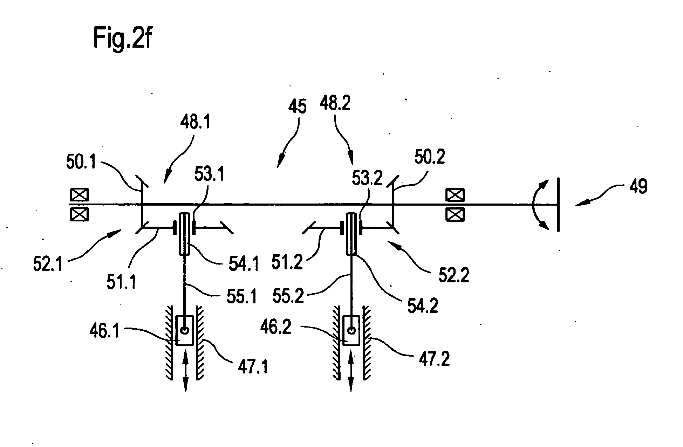

Die Figuren 2b und 2c verdeutlichen jeweils eine Ansicht von Rechts und von Links. Diese verdeutlichen die Anordnung von Verteilerkanal 2, sowie der Verbindungskanäle 3 und 4 und des Funktionskanals 5 in einer Ebene E1. Erkennbar ist ferner die Querschnittsgeometrie des Trocknungsschwertes 38. Dieses ist flügelartig ausgeführt und durch eine, Austrittsöffnungen 6.1 bis 6.n tragende Oberfläche 39 charakterisiert. Das Schwert 38 ist dabei hinsichtlich der Außenkontur wenigstens durch eine erste obere Fläche 42, welche in Einbaulage zum Verteilerkanal 2 gerichtet ist und eine zweite untere Fläche 43, welche in Einbaulage vom Verteilerkanal 2 weggerichtet ist, charakterisiert. Die Austrittsöffnungen 6.1 bis 6.n können dabei sowohl an der in Einbaulage zum Verteilerkanal 2 ausgerichteten bzw. weisenden oberen Fläche 42, und/oder an der vom Verteilerkanal 2 weggerichteten Fläche 43 angeordnet sein. Die einzelne obere Fläche 42 und die untere Fläche 43 ist dabei durch wenigstens zwei Teilflächen charakterisiert, die aneinander anschließen und durch unterschiedliche Geometrien charakterisiert sein können und in einem Winkel zueinander oder aneinander anschließend angeordnet sind. Im dargestellten Fall weist das Schwert 38 vorzugsweise einen hinsichtlich einer Horizontalebene, welche durch die Längsachse A5 und eine Senkrechte zu dieser charakterisiert ist, symmetrischen Aufbau auf. Die einzelnen Flächen 42 und 43 weisen jeweils zwei Teilflächen 42.11, 42.22 und 43.11, 43.22 auf. Die Teilflächen 42.11 und 43.11 sind jeweils im Querschnitt betrachtet gekrümmt ausgeführt, während die Teilflächen 42.22 und 43.22 einen im wesentlichen ebenen Verlauf aufweisen, wobei der Übergang, d.h. die Verbindung zwischen oberer und unterer Fläche 42 und 43 weich, d.h. gerundet ausgeführt ist. Wenigstens ein Teilbereich der Flächen - obere und/oder untere Fläche 42, 43 - trägt Austrittsöffnungen 6.1 bis 6.n. Erkennbar ist ferner eine Stelleinrichtung 45 zur Verschiebung des Funktionskanals 5 entlang der Längsachsen A3, A4 der Verbindungskanäle 3 und 4. Der Funktionskanal 5 ist dazu an seinen beiden Enden in einem Schieber 46.1, 46.2 gelagert. Dieser ist in einer parallel zur Längsachse A3 bzw. A4 der Verbindungskanäle 3, 4 ausgerichteten Führung 47.1 bzw. 47.2 verschiebbar geführt. Die Stelleinrichtung 45.1 bzw. 45.2 umfasst eine Übertragungseinheit 48.1 bzw. 48.2, welcher die über eine Betätigungseinrichtung 49 vorgegebenen Drehwinkel in eine Verstellbewegung in Längsrichtung der Achsen A3 und A4 umwandelt. Der Aufbau und die Funktionsweise sind anhand eines Schemas in Figur 2f beschrieben.

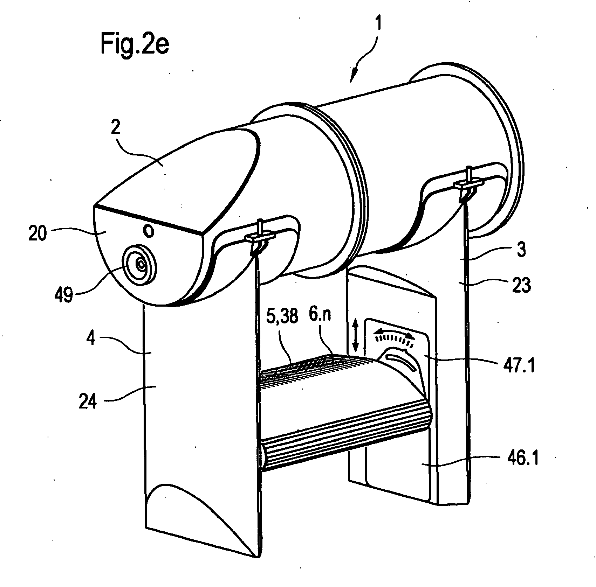

Die Figur 2d verdeutlicht eine Perspektivansicht gemäß Figur 2a und die Figur 2e eine Ansicht von Oben, während Figur 2f das Wirkprinzip einer beispielhaften mechanischen Stelleinrichtung 45 wiedergibt.

Bezüglich der Realisierung der Verdrehbarkeit und der Verschiebbarkeit bestehen eine Vielzahl von Möglichkeiten. Im einfachsten Fall werden mechanische Lösungen gewählt. Das Trocknungsschwert 38 bzw. der Funktionskanal 5 ist drehbar gelagert. Die Lagerung erfolgt jeweils drehbar an einem Schieber 46.1, 46.2 der in einer einem Stutzen 23 und 24 zugeordneten Führung 47.1, 47.2 verschiebbar gelagert ist. Über die Betätigungseinrichtung 49 wird ein Verdrehwinkel vorgegeben, der einem Verstellweg s entspricht. Gemäß Figur 2f wird diese als Winkel vorgegeben Sollgröße sₛₒₗₗ über eine Übertragungseinheit in den Verstellweg s umgewandelt. Dazu ist ein Winkeltrieb 52.1, 52.2 vorgesehen, dessen Ritzel 50.1, 50.2 drehfest mit der Betätigungseinrichtung 49 verbunden sind und die mit einem Ausgangszahnrad 51.1, 51.2 kämmen, wobei das einzelne Ausgangszahnrad jeweils parallel zur Längsachse A4, A3 angeordnet ist und ein Innengewinde 53.1, 53.2 trägt, welches mit einem Außengewinde 54.1, 54.2 an einem Verbindungselement 55.1, 55.2 in Form eines Stabes zusammenwirkt. Das Verbindungselement 55.1, 55.2 ist mit dem Schieber 46.1, 46.2 verbunden. Die Korrelation zwischen Verdrehwinkel und Verschiebeweg s wird durch die Steigung des Gewindes bestimmt.

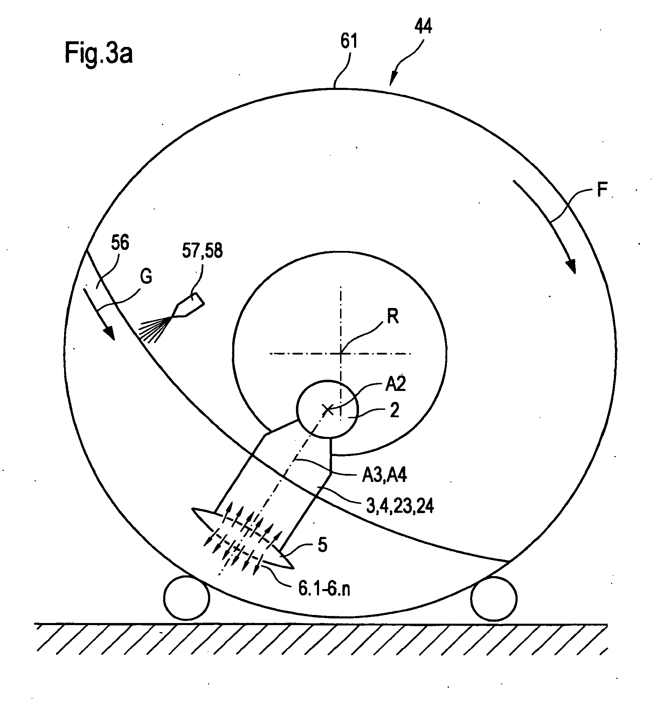

Die Figur 3a verdeutlicht anhand einer stark schematisierten Darstellung eine erfindungsgemäß gestaltete Vorrichtung 44 zum Behandeln körnigen Gutes mit einer erfindungsgemäßen Einrichtung 1. Die Abluftführung ist in dieser Ausführung nicht dargestellt. Diese kann auf herkömmliche Art und Weise, wie aus dem Stand der Technik vorbekannt, erfolgen. Die Vorrichtung 44 umfasst eine um eine Rotationsachse R drehbar gelagerte Trommel 61, in welcher eine vorgegebene Menge an zu behandelnden Gut 56 enthalten ist. Die Einrichtung 1 ist innerhalb der Trommel 61 parallel zur Rotations- bzw. Drehachse derselben angeordnet und steht mit einer außerhalb der Trommel 61 angeordneten und hier nicht dargestellten Gasbereitstellungseinheit in Verbindung. Die Längsachsen A2 und A5 von Verteilerkanal 2 und Funktionskanal 5 sind ebenfalls parallel zur Rotationsachse R angeordnet. Die beiden Stutzen 23, 24, welche die Verbindungskanäle 3 und 4 bilden, erstrecken sich senkrecht vom Verteilerkanal 2. Die Anordnung der Einrichtung 1 erfolgt dabei in Einbaulage der Trommel 61 vorzugsweise im unteren Bereich, d.h. unterhalb einer Horizontalebene, welche durch die Rotationsachse der Trommel 61 und eine Senkrechte dazu in Horizontalrichtung charakterisiert ist. Denkbar ist auch eine Anordnung in radialer Richtung um die Rotationsachse R. Die Anordnung des Verteilerkanals 2 erfolgt vorzugsweise parallel zur Rotationsachse und entweder in einer Vertikalebene mit dieser mit oder ohne Versatz in Vertikalrichtung oder mit Versatz sowohl in Vertikal- als auch Horizontalrichtung in Einbaulage der Trommel 61 betrachtet, gegenüber dieser. Die Verbindungskanäle 3 und 4 erstrecken sich dabei in einem Winkel gegenüber dieser und in einem Winkel gegenüber der durch die Rotationsachse R und eine Senkrechte in Vertikalrichtung beschreibbare Vertikalebene. Vorzugsweise erstrecken sich die Verbindungskanäle 3, 4 in radialer Richtung vom Verteilerkanal 2 und in radialer Richtung von der Rotationsachse R in Richtung der Trommelinnenwandung und ermöglichen ein Eintauchen oder zumindest in Wirkverbindung bringen des Funktionskanals 5 mit seinen Auslässen 6.1 bis 6.n mit dem Gut. Andere Ausrichtungen sind denkbar. Die Vorrichtung 44 umfasst ferner eine Einrichtung 57 zum Auftragen eines Mediums auf das Gut 56, insbesondere eine Einrichtung 57 zum Befeuchten, Besprühen oder Beschichten. Im dargestellten Fall beispielhaft in Form von Düsen 58, die mit entsprechenden, sich in die Trommel 61 erstreckenden und hier nicht dargestellten Mediumversorgungs- und Führungseinrichtungen außerhalb der Trommel 61 koppelbar sind und entweder als Schlitzdüsen oder in einer Vielzahl einzelner Düsenelemente, die axial beabstandet zueinander angeordnet sind, vorliegen. Dabei wird über die Einrichtung 57 von oben her Befeuchtungs- oder Beschichtungsmedium auf eine obere Schicht des Gutes 56 aufgetragen, während sich die Trommel 61 in Richtung des Pfeils F dreht, eine untere Schicht nach oben mitnimmt und dadurch bewirkt, dass die obere Schicht des Gutes in Richtung des Pfeils G unter einem Winkel gegen die Waagerechte, vorzugsweise von ca. 45° abströmt. In diese abwärtsströmende obere Schicht des Gutes 56 taucht der Funktionskanal 5 bzw. das diesen bildende Element und beaufschlagt über die Auslässe 6.1 bis 6.n das Gut 56 mit Trocknungsgas, vorzugsweise Trocknungsluft. Dabei ist der Funktionskanal 5 der Einrichtung 57 nachgeordnet.

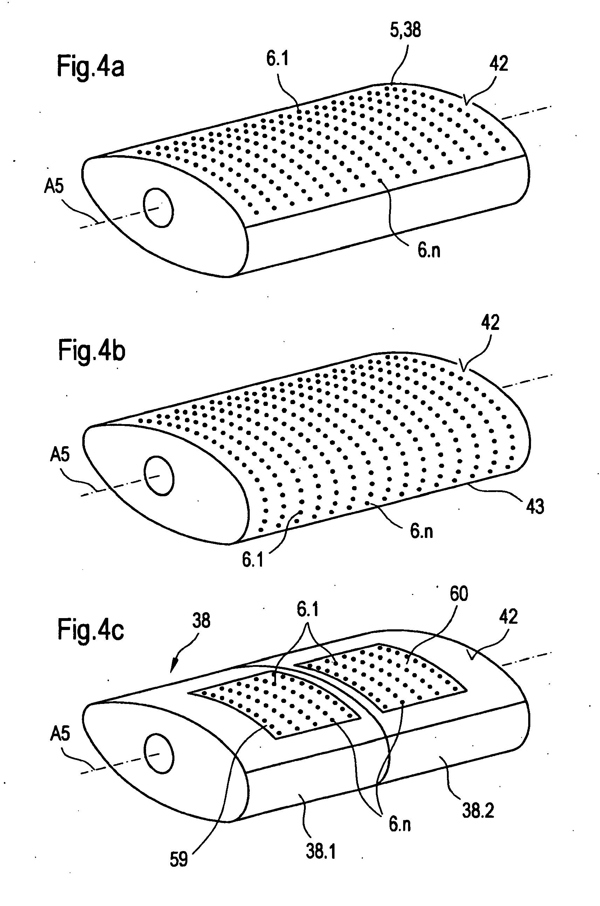

In Figur 3a wird das Wirkprinzip für eine Ausführung mit an beiden Seiten des Funktionskanals 5 angeordneten Auslässen 6.1 bis 6.n, wie beispielhaft in Figur 4b dargestellt, wiedergegeben. Der Funktionskanal 5 ist hier in seiner Lage gegenüber dem Verteilerkanal 2 in radialer Richtung ortsfest, d.h. nicht verschiebbar. Eine Verdrehbarkeit um seine Längsachse A5 ist denkbar, jedoch nicht erforderlich. Bei dieser Lösung wird unabhängig vom Befüllungsgrad der Trommel 61 sowohl die obere Schicht des Gutes 56, welche im radial inneren Bereich zurückströmt und die untere, an der Innenwandung der Trommel 61 nach oben mitgenommene Schicht des Gutes 56 großflächig einem Trocknungsgas ausgesetzt. Die Schichtdicke des über den Funktionskanal 5 fließenden Gutes 56 ist unterschiedlich und abhängig von der Füllmenge. Denkbar, hier jedoch nicht dargestellt, ist die Möglichkeit der Verfahr- oder Verschiebbarkeit des Funktionskanals gegenüber der Rotationachse der Trommel 61 bzw. dem Verteilerkanal 2 in radialer Richtung.

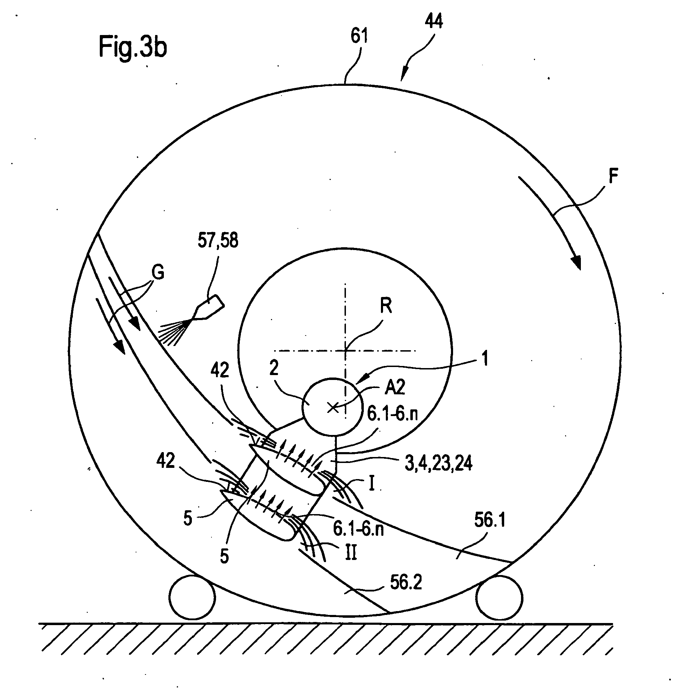

Demgegenüber verdeutlicht Figur 3b eine weitere Ausführungsform mit verstellbarem Funktionskanal 5, wie beispielhaft in den Figuren 1c und 2 wiedergegeben. Der Funktionskanal 5 weist dabei nur an seiner zum Verteilerkanal 2 gerichteten Seite, insbesondere oberen Fläche 42 Auslassöffnungen 6.1 bis 6.n auf. Der Funktionskanal 5 ist parallel zu den Längsachsen A3, A4 der Verbindungskanäle 3, 4 an diesen verschiebbar. Figur 3b verdeutlicht dabei die Verhältnisse für unterschiedliche Befüllgrade. 56.1 verdeutlicht einen maximalen Befüllungsgrad, bei welchem der Funktionskanal 5 in der Funktionsstellung I angeordnet ist bzw. in diese verbracht wird. 56.2 verdeutlicht die Schichtdicke des Gutes bei minimaler Befüllmenge. Der Funktionskanal 5 befindet sich dann in der Funktionsstellung II. Aufgrund der Höhenverstellbarkeit des Funktionskanals 5 und der vorzugsweise zusätzlichen Verdrehbarkeit kann der Funktionskanal 5 einen Volumenanstieg ausgleichen und das Gut 56 fließt in jeder der Funktionsstellungen I, II und der dazwischen liegenden Stellungen immer mit gleicher Schichtdicke über den Funktionskanal 5. Die Verstellbarkeit kann dabei aktiv während des Behandlungsprozesses bei Änderung der Befüllmenge oder aber jeweils vor Befüllung erfolgen.

Figuren 4a bis 4c verdeutlichen beispielhaft mögliche Anordnungen der Auslässe 6.1 bis 6.n am Funktionskanal 5 und Ausführungen des Funktionskanals 5.

Figur 4a verdeutlicht eine Ausführung eines Schwertes 38 mit nur an den die Oberseite bildenden Flächenbereichen 42 angeordneten Auslässen 6.1 bis 6.n.

Figur 4b verdeutlicht eine Ausführung des den Funktionskanal 5 bildenden Schwertes 38 mit an der die Ober- und Unterseite bildenden Flächen 42, 43 angeordneten Auslässen 6.1 bis 6.n.

Bei beiden Ausführungen erfolgt die Anordnung der Auslässe 6.1 bis 6.n vorzugsweise über die gesamte Erstreckung des Funktionskanals 5 in Längsrichtung, d.h. entlang der Längsachse A5.

Demgegenüber verdeutlicht Figur 4c eine weitere Ausführungsform eines den Funktionskanal 5 tragenden oder bildenden Schwertes 38, welches teleskopartig aufgebaut ist und damit eine Anpassung seiner Länge ermöglicht. Dazu ist das Schwert 38 wenigstens zweiteilig ausgeführt, wobei die einzelnen Teilelemente 38.1, 38.2 gegeneinander verschiebbar sind, wobei das Teilelement 38.1 wenigstens teilweise in 38.2 einschiebbar ist. Beide Teilelemente 38.1 und 38.2 sind dazu im Verschiebungsbereich mit unterschiedlichen Abmessungen ausgeführt. Ferner ist die Verbindung zwischen beiden im wesentlichen luftdicht ausgeführt. Die Auslässe 6.1 bis 6.n sind hier jeweils in einer Mehrzahl, hier beispielhaft zwei Flächenbereichen 59 und 60 konzentriert. Diese können lösbar mit dem den Funktionskanal 5 bildenden Grundelement verbunden sein, wobei dann im Fall einer vollständigen Überdeckung nach dem Zusammenschieben der Flächenbereich 59 des dann inneren Elementes 59 entfernt wird und der Austritt allein über den Flächenbereich 60 realisiert wird.

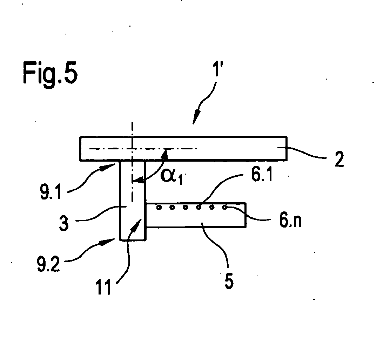

Die Figur 5 verdeutlicht in schematisch vereinfachter Darstellung den Grundaufbau einer erfindungsgemäß ausgeführten Einrichtung 1' für den Einsatz in Vorrichtungen zur Behandlung körnigen Gutes durch Trocknen, Filmcoaten oder Beschichten, insbesondere für Vorrichtungen zur Behandlung pharmazeutischer Partikel gemäß dem zweiten Lösungsansatz. Diese umfasst einen zentralen Verteilkanal 2, der mit einer hier nicht dargestellten Zuluftversorgungseinrichtung beim Einsatz in den vorgenannten Vorrichtungen verbindbar ist und von welchem sich ein Verbindungskanal 3 in einem Winkel α1, vorzugsweise 90° erstreckt. Es ist ein Zusammenführungskanal, welcher als eigentlicher Funktionskanal 5 in Form des Auslasskanals fungiert vorgesehen, der beabstandet zum Verteilerkanal 2 angeordnet ist und mit dem Verbindungskanal 3 verbunden ist. Dieser umfasst einen Einlassbereich 11 und eine Vielzahl von Austrittsöffnungen 6.1 bis 6.n für die über den Kanal 3 in diesen mündende Zuluft. Dadurch wird ein Austritt über einen größeren Flächenbereich gewährleistet. Entsprechend der Anordnung und der Kopplung der einzelnen Kanäle miteinander können mehrere Grundvarianten unterschieden werden. Alle Varianten können dabei mit fester Zuordnung hinsichtlich der Lage zwischen dem Verteilerkanal 2 und dem Funktionskanal 5 oder aber veränderbarer Zuordnung ausgeführt werden. Zuordnungsänderungen sind im wesentlichen durch die mögliche Verdrehbarkeit des Funktionskanals 5 um seine Achse A5, insbesondere Lagerachse und/oder die Verschiebbarkeit gegenüber dem Verteilerkanal 2 unter Veränderung des Abstandes h in Vertikalrichtung zum Verteilerkanal 2 charakterisiert. Bei allen Ausführungen bestehen für die gewählten Querschnittsgeometrien der einzelnen Kanäle, insbesondere des Verbindungskanals 3 sowie des Verteilerkanals 2 und des Funktionskanals 5 keine Beschränkungen. Im dargestellten Fall erstreckt sich der Funktionskanal 5 vom Verbindungskanal 3 weg und ist vorzugsweise an diesem fliegend gelagert. Denkbar wäre auch eine mittige Ankoppelung des Verbindungskanals 3 am Funktionskanal 5. Dabei erfolgt eine Strömungsumkehr oder zumindest Umlenkung zwischen Verteilerkanal und den Auslässen 6.n. Der Funktionskanal 5 kann ortsfest gegenüber dem Verteilerkanal 2 angeordnet sein oder aber über die bereits in den vorherigen Figuren beschriebenen Möglichkeiten in seiner Lage gegenüber dem Verteilerkanal 2 verändert werden, d.h. in der Höhe verstellt und/oder verdreht werden.

Alle beschriebenen Ausführungen sind Beispiele, welche den Schutzbereich nicht beschränken. Die möglichen Ausführungen der Einzelelemente können auch miteinander kombiniert werden.

### Bezugszeichenliste

- 1, 1': Einrichtung
- 2: zentraler Verteilerkanal
- 3: Kanal
- 4: Kanal
- 5: Funktionskanal
- 6.1, 6.n: Austrittsöffnungen
- 7: Hohlkörper
- 8: rohrförmiges Element
- 9.1: erster Endbereich
- 9.2: zweiter Endbereich
- 10.1: erster Endbereich
- 10.2: zweiter Endbereich
- 11: Einlassbereich
- 12: Einlassbereich
- 13: Flanschkupplungen
- 14: Teilelement
- 15: Teilelement
- 16: Grundrohr
- 17: Aufsatzrohr
- 18: Innenwandung
- 19: Stirnseite
- 20: Stirnseite
- 21: Öffnung
- 22: Außenumfang
- 23: Stutzen
- 24: Stutzen
- 25: Innenraum
- 26: Innenraum
- 27: Ende des Stutzens
- 28: Ende des Stutzens
- 29: Flansch
- 30: Flansch
- 31: Öffnung
- 32: Öffnung
- 33: Austritt am Element 8
- 34: Austritt am Element 8
- 35: Mündungsbereich
- 36: Mündungsbereich
- 37: Hohlkörper
- 38: Schwert
- 40: Endbereich
- 41: Endbereich
- 42: obere Fläche
- 43: untere Fläche
- 44: Vorrichtung zur Behandlung körnigen Gutes
- 45: Stelleinrichtung
- 46.1, 46.2: Schieber
- 47.1,47.2: Führung
- 48.1, 48.2: Übertragungseinheit
- 49: Betätigungseinrichtung
- 50.1, 50.2: Ritzel
- 51.1, 51.2: Ausgangszahnrad
- 52.1, 52.2: Winkeltrieb
- 53.1, 53.2: Innengewinde .
- 54.1, 54.2: Außengewinde
- 55.1, 55.2: Verbindungselement
- 56: Gut
- 57: Einrichtung zum Auftragen eines Mediums
- 58: Düse
- 59: Flächenbereich
- 60: Flächenbereich
- 61: Trommel

## Patentansprüche

1. Einrichtung (1) zur Führung eines Gases für Vorrichtungen (44) zum Behandeln körnigen Gutes (56) durch Trocknen, Filmcoaten oder Beschichten, insbesondere Zulufteinheit, umfassend einen zentralen Verteilerkanal (2), der mit einer Gasbereitstellungseinrichtung verbindbar ist und mit von diesem beabstandet angeordneten und in axialer Richtung zueinander beabstandet angeordneten Auslässen (6.1, 6.n) für das Gas über Verbindungskanäle (3, 4) verbunden ist, **gekennzeichnet durch** die folgenden Merkmale:
- mit einem eine Vielzahl von Auslässen (6.1, 6.n) aufweisenden und zum Verteilerkanal (2) beabstandet angeordneten Funktionskanal (5);
- der Funktionskanal (5) umfasst wenigstens zwei zueinander axial beabstandet angeordnete Einlassbereiche (11, 12), wobei jeder der Einlassbereiche (11, 12) mit dem Verteilerkanal (2) über einen Verbindungskanal (3, 4) verbunden ist;
- die Auslässe (6.1, 6.n) sind in axialer Richtung am Funktionskanals (5) betrachtet zwischen der Mündung zweier in axialer Richtung benachbart angeordneter Verbindungskanäle (3, 4) in den Funktionskanal (5) angeordnet, und der Funktionskanal (5) gegenüber dem Verteilerkanal (2) in seiner Lage veränderbar ist.

2. Einrichtung (1') zur Führung eines Gases für Vorrichtungen (44) zum Behandeln körnigen Gutes (56) durch Trocknen, Filmcoaten oder Beschichten, insbesondere Zulufteinheit, umfassend einen zentralen Verteilerkanal (2), der mit einer Gasbereitstellungseinrichtung verbindbar ist und mit von diesem beabstandet angeordneten und in axialer Richtung zueinander beabstandet angeordneten Auslässen (6.1, 6.n) für das Gas über einen Verbindungskanal (3) verbunden ist, **gekennzeichnet durch** die folgenden Merkmale:
- mit einem eine Vielzahl von Auslässen (6.1, 6.n) aufweisenden und zum Verteilerkanal (2) beabstandet angeordneten Funktionskanal (5);
- der Funktionskanal (5) umfasst einen Einlassbereich (11), der mit dem Verteilerkanal (2) über den Verbindungskanal (3) verbunden ist, wobei der Verbindungskanal (3) und der Funktionskanal (5) in einem Winkel zueinander angeordnet sind, und der Funktionskanal (5) gegenüber dem Verteilerkanal (2) in seiner Lage veränderbar ist.

3. Einrichtung (1') nach Anspruch 2, **dadurch gekennzeichnet, dass** der Funktionskanal (5) fliegend am Verbindungskanal (3) gelagert ist.

4. Einrichtung (1, 1') nach einem der Ansprüche 1 bis 3, **dadurch gekennzeichnet, dass** die Auslässe (6.1, 6.n) wenigstens an dem zum Verteilerkanal (2) weisenden Bereich der Außenfläche (42) des Funktionskanals (5) angeordnet sind.

5. Einrichtung (1, 1') nach einem der Ansprüche 1 bis 4, **dadurch gekennzeichnet, dass** die Auslässe (6.1, 6.n) an dem vom Verteilerkanal (2) weggerichteten Bereich der Außenfläche (43) des Funktionskanals (5) angeordnet sind.

6. Einrichtung (1, 1') nach einem der Ansprüche 1 bis 5, **dadurch gekennzeichnet, dass** die Auslässe (6.1, 6.n) wenigstens einen vordefinierten Flächenbereich (59, 60) am Außenumfang des Funktionskanals (5) überdecken.

7. Einrichtung (1, 1') nach Anspruch 6, **dadurch gekennzeichnet, dass** die einzelnen Auslässe (6.1, 6.n) durch einen Auslassquerschnitt durch Abmaße im Bereich von 0,1 bis 1 mm, bevorzugt 0,2 bis 0,6 mm, insbesondere etwa 0,5 mm charakterisiert sind und mit einem vordefinierten Abstand im Bereich von 0,5 bis 2 mm, bevorzugt 0,8 bis 1,5 mm, insbesondere etwa 1 mm zueinander in axialer Richtung und senkrecht dazu zueinander angeordnet sind.

8. Einrichtung (1, 1') nach einem der Ansprüche 1 bis 7, **dadurch gekennzeichnet, dass** die Auslässe (6.1, 6.n) in axialer Richtung betrachtet wenigstens über einen Teilbereich der axialen Erstreckung des Funktionskanals (5) angeordnet sind.

9. Einrichtung (1, 1') nach einem der Ansprüche 1 bis 8, **dadurch gekennzeichnet, dass** der Verteilerkanal (2) und der Funktionskanal (5) parallel zueinander angeordnet sind.

10. Einrichtung (1, 1') nach einem der Ansprüche 1 bis 9, **dadurch gekennzeichnet, dass** der Verteilerkanal (2) und der Funktionskanal (5) in einem Winkel zueinander angeordnet sind.

11. Einrichtung (1, 1') nach einem der Ansprüche 1 bis 10, **dadurch gekennzeichnet, dass** der Verteilerkanal (2) und der einzelne Verbindungskanal (3, 4) senkrecht zueinander angeordnet sind.

12. Einrichtung (1, 1') nach einem der Ansprüche 1 bis 11, **dadurch gekennzeichnet, dass** der Funktionskanal (5) und der einzelne Verbindungskanal (3, 4) senkrecht zueinander angeordnet sind.

13. Einrichtung (1, 1') nach einem der Ansprüche 1 bis 12, **dadurch gekennzeichnet, dass** der Verteilerkanal (2), der Funktionskanal (5) und der einzelne Verbindungskanal (3, 4) jeweils durch eine beliebige Querschnittsgeometrie charakterisiert sind.

14. Einrichtung (1, 1') nach einem der Ansprüche 1 bis 13, **dadurch gekennzeichnet, dass** der Funktionskanal (5) im Querschnitt betrachtet flügelartig ausgeführt ist und von einer durch abgerundete Übergänge miteinander verbundene Oberseite (42) und Unterseite (43) gebildet wird, wobei die Ober- und die Unterseite (42, 43) im Querschnitt betrachtet jeweils durch die Aneinanderreihung unterschiedlich ausgestalteter und ausgerichteter Flächenbereiche (42.1, 42.2, 43.1, 43.2) charakterisiert ist.

15. Einrichtung (1, 1') nach Anspruch 14, **dadurch gekennzeichnet, dass** der Funktionskanal (5) im Querschnitt bezüglich einer Ebene symmetrisch ausgeführt ist.

16. Einrichtung (1, 1') nach einem der Ansprüche 1 bis 15, **dadurch gekennzeichnet, dass** der Funktionskanal (5) um seine Längsachse (A5) drehbar ist.

17. Einrichtung (1, 1') nach einem der Ansprüche 1 bis 16, **dadurch gekennzeichnet, dass** der Funktionskanal (5) parallel zu dem einzelnen Verbindungskanal (3) oder Verbindungskanälen (3, 4) verschiebbar ist.

18. Einrichtung (1, 1') nach einem der Ansprüche 1 bis 17, **dadurch gekennzeichnet, dass** dem Funktionskanal (5) wenigstens eine Stelleinrichtung (45) zur Lageänderung zugeordnet ist, wobei die Stelleinrichtung nach einem der nachfolgenden Wirkprinzipien fungiert:
- mechanisch
- hydraulisch
- pneumatisch
- elektronisch
- eine Kombination aus vorgenannten.

19. Einrichtung (1, 1') nach Anspruch 18, **dadurch gekennzeichnet, dass** die Stelleinrichtung (45) im Verteilerkanal (2) und dem oder den Verbindungskanälen (3, 4) integriert ist.

20. Einrichtung (1, 1') nach Anspruch 18, **dadurch gekennzeichnet, dass** die Stelleinrichtung (45) außerhalb von Verteilerkanal (2) und dem Verbindungskanal (3) oder den Verbindungskanälen (3, 4) angeordnet ist.

21. Einrichtung (1, 1') nach eine der Ansprüche 1 bis 20, **dadurch gekennzeichnet, dass** der einzelne Verbindungskanal (3, 4) in seiner Länge unveränderlich ist.

22. Einrichtung (1, 1') nach einem der Ansprüche 1 bis 21, **dadurch gekennzeichnet, dass** der Funktionskanal (5) in seinem Einlassbereich oder Einlassbereichen (11, 12) jeweils in einem Schieber (46.1, 46.2) gelagert ist, der an den die Verbindungskanäle (3, 4) bildenden Elementen (23, 24) in einer Führung (47.1, 47.2) führbar ist, wobei in jeder Position des Schiebers (46.1, 46.2) gegenüber der Führung (47.1, 47.2) der Funktionskanal (5) mit dem Verteilerkanal (2) strömungsführend verbunden ist.

23. Einrichtung (1, 1') nach einem der Ansprüche 1 bis 21, **dadurch gekennzeichnet, dass** der Verbindungskanal teleskopierbar ausgeführt ist.

24. Einrichtung (1, 1') nach einem der Ansprüche 1 bis 23, **dadurch gekennzeichnet, dass** eine Betätigungseinrichtung (49) vorgesehen ist, an der ein Sollwert für eine einen Verstellweg s und/oder einen Verdrehwinkel wenigstens mittelbar charakterisierende Größe vorgebbar ist und die mit einer Übertragungseinheit verbunden ist, die die Sollgröße in einen Stellgröße umsetzt.

25. Einrichtung (1, 1') nach Anspruch 24, **dadurch gekennzeichnet, dass** die Übertragungseinheit ein Getriebe umfasst, welches eine Drehbewegung in eine Längsbewegung umwandelt.

26. Einrichtung (1, 1') nach einem der Ansprüche 1 bis 25, **dadurch gekennzeichnet, dass** der Funktionskanal (5) teleskopierbar ausgeführt ist.

27. Einrichtung (1, 1') nach einem der Ansprüche 1 bis 26, **dadurch gekennzeichnet, dass** der Verbindungskanal (3, 4) von einem einen Hohlkörper bildenden Element, welches jeweils mit dem Verteilerkanal (2) und dem Verbindungskanal über den Öffnungsbereich in strömungsführender Verbindung steht, und sich vom Verteilerkanal (2) wegerstreckt, gebildet wird.

28. Einrichtung (1, 1') nach einem der Ansprüche 1 bis 27, **dadurch gekennzeichnet, dass** der Funktionskanal (5) von einem einen Hohlkörper bildenden Element gebildet wird, dessen Außenumfang wenigsten in Teilbereichen eine gelochte Oberfläche, insbesondere eine Perforation aufweist.

29. Einrichtung (1, 1') nach Anspruch 28, **dadurch gekennzeichnet, dass** der den Funktionskanal (5) bildende Hohlkörper wenigstens teilweise aus einem Lochblech mit Stützkonstruktion oder einer perforierten Metallfolie besteht.

30. Einrichtung (1, 1') nach einem der Ansprüche 1 bis 29, **dadurch gekennzeichnet, dass** die die einzelnen Kanäle (2, 3, 4, 5) bildenden Elemente aus Metall, Kunststoff oder inertem Material, vorzugsweise Edelstahl bestehen.

31. Einrichtung (1, 1') nach Anspruch 30, **dadurch gekennzeichnet, dass** die die einzelnen Kanäle (2, 3, 4, 5) bildenden oder tragenden Elemente aus Edelstahlbauteilen gebildet sind, welche jeweils lösbar oder unlösbar miteinander verbunden sind.

32. Einrichtung (1, 1') nach einem der Ansprüche 1 bis 31, **dadurch gekennzeichnet, dass** der Funktionskanal (5) mehrteilig ausgeführt ist und die die Auslässe (6.1, 6.n) bildenden Flächenbereiche (59, 60) lösbar mit dem den Funktionskanal (5) bildenden Grundelement verbunden sind.

33. Vorrichtung (44) zum Behandeln körnigen Gutes durch Trocknen, Filmcoaten oder Beschichten, insbesondere zum Behandeln pharmazeutischer Partikel
mit einer drehbar um eine Achse gelagerten und mit dem Gut (56) befüllbaren Trommel (61);
mit einer in der Trommel (61) angeordneten Einrichtung (1, 1') zur Führung eines Gases nach einem der Ansprüche 1 bis 32.

34. Vorrichtung (44) nach Anspruch 33, **dadurch gekennzeichnet, dass** der Funktionskanal (5) parallel zur Rotationsachse (R) der Trommel (61) angeordnet ist.

35. Vorrichtung (44) nach Anspruch 33 oder 34, **durch gekennzeichnet, dass** der Einrichtung (1, 1') eine Einrichtung (57) zum Auftragen von Medium vorgeordnet ist.

36. Verwendung einer Vorrichtung nach einem der Ansprüche 33 bis 35 zur Herstellung von Dabigatran etexilate Pellets oder Dipyridamol Retardformen.

## Claims

1. Installation (1) for guiding a gas for apparatus (44) for treating granular products (56) by drying, film-coating or coating, especially an incoming air unit, comprising a central distribution channel (2) that can be connected to a gas supply device and is connected, via connection channels (3, 4), to axially spaced outlets (6.1, 6.n) for the gas that are spaced from said channel, **characterised by** the following features:
- having a functional channel (5) that comprises a plurality of outlets (6.1, 6.n) and is spaced from the distribution channel (2);
- the functional channel (5) comprises at least two inlet regions (11, 12) axially spaced from one another, each of the inlet regions (11, 12) being connected to the distribution channel (2) through a connection channel (3, 4);
- the outlets (6.1, 6.n) are arranged, viewed in the axial direction at the functional channel (5), between the mouths of two axially adjacent connection channels (3, 4) into the functional channel (5), and the functional channel (5) is variable in its position relative to the distribution channel (2).

2. Installation (1') for guiding a gas for apparatus (44) for treating granular products (56) by drying, film-coating or coating, especially an incoming air unit, comprising a central distribution channel (2) that can be connected to a gas supply device and is connected, via a connection channel (3), to axially spaced outlets (6.1, 6.n) for the gas that are spaced from said channel, **characterised by** the following features:
- having a functional channel (5) that comprises a plurality of outlets (6.1, 6.n) and is spaced from the distribution channel (2);
- the functional channel (5) comprises an inlet region (11) connected to the distribution channel (2) through the connection channel (3), the connection channel (3) and the functional channel (5) being arranged at an angle to one another, and the functional channel (5) is variable in its position relative to the distribution channel (2).

3. Installation (1') according to Claim 2, **characterised in that** the functional channel (5) is mounted in an overhung position on the connection channel (3).

4. Installation (1, 1') according to one of Claims 1 to 3, **characterised in that** the outlets (6.1, 6.n) are arranged at least on the part of the outer surface (42) of the functional channel (5) facing the distribution channel (2).

5. Installation (1, 1') according to one of Claims 1 to 4, **characterised in that** the outlets (6.1, 6.n) are arranged on the part of the outer surface (43) of the functional channel (5) directed away from the distribution channel (2).

6. Installation (1, 1') according to one of Claims 1 to 5, **characterised in that** the outlets (6.1, 6.n) cover at least one predefined surface zone (59, 60) on the outer periphery of the functional channel (5).

7. Installation (1, 1') according to Claim 6, **characterised in that** the individual outlets (6.1, 6.n) are **characterised by** an outlet cross section with dimensions in the range from 0.1 to 1 mm, preferably 0.2 to 0.6 mm, especially about 0.5 mm and are arranged at a predefined spacing in the range from 0.5 to 2 mm, preferably 0.8 to 1.5 mm, especially about 1 mm from one another in the axial direction and perpendicularly thereto.

8. Installation (1, 1') according to one of Claims 1 to 7, **characterised in that** the outlets (6.1, 6.n) viewed in the axial direction are arranged at least over part of the axial extent of the functional channel (5).

9. Installation (1, 1') according to one of Claims 1 to 8, **characterised in that** the distribution channel (2) and the functional channel (5) are arranged parallel to one another.

10. Installation (1, 1') according to one of Claims 1 to 9, **characterised in that** the distribution channel (2) and the functional channel (5) are arranged at an angle to one another.

11. Installation (1, 1') according to one of Claims 1 to 10, **characterised in that** the distribution channel (2) and the single connection channel (3, 4) are arranged perpendicularly to one another.

12. Installation (1, 1') according to one of Claims 1 to 11, **characterised in that** the functional channel (5) and the single connection channel (3, 4) are arranged perpendicularly to one another.

13. Installation (1, 1') according to one of Claims 1 to 12, **characterised in that** the distribution channel (2), the functional channel (5) and the single connection channel (3, 4) are each **characterised by** having any desired cross-sectional geometry.

14. Installation (1, 1') according to one of Claims 1 to 13, **characterised in that** the functional channel (5) is wing-like, viewed in cross-section, and is formed by a top side (42) and bottom side (43) joined together by rounded transitions, the top and bottom sides (42, 43) viewed in cross-section each being **characterised by** the side-by-side arrangement of differently configured and aligned surface zones (42.1, 42.2, 43.1, 43.2).

15. Installation (1, 1') according to Claim 14, **characterised in that** the functional channel (5) is symmetrically constructed in cross-section relative to a plane.

16. Installation (1, 1') according to one of Claims 1 to 15, **characterised in that** the functional channel (5) is rotatable about its longitudinal axis (A5).

17. Installation (1, 1') according to one of Claims 1 to 16, **characterised in that** the functional channel (5) is movable parallel to the individual connection channel (3) or connection channels (3, 4).

18. Installation (1, 1') according to one of Claims 1 to 17, **characterised in that** at least one positioning device (45) is associated with the functional channel (5) for changing position, the positioning device operating according to one of the following principles:
- mechanical
- hydraulic
- pneumatic
- electronic
- a combination of the above.

19. Installation (1, 1') according to Claim 18, **characterised in that** the positioning device (45) is integrated in the distribution channel (2) and the connection channel or channels (3, 4).

20. Installation (1, 1') according to Claim 18, **characterised in that** the positioning device (45) is arranged outside the distribution channel (2) and connection channel (3) or connection channels (3, 4).

21. Installation (1, 1') according to one of Claims 1 to 20, **characterised in that** the single connection channel (3, 4) is invariable in its length.

22. Installation (1, 1') according to one of Claims 1 to 21, **characterised in that** the functional channel (5) in its inlet region or inlet regions (11, 12) is mounted in a slide (46.1, 46.2) which can be guided on the elements (23, 24) that form the connection channels (3, 4) in a guide (47.1, 47.2), while in every position of the slide (46.1, 46.2) relative to the guide (47.1, 47.2) the functional channel (5) is connected in flow-carrying manner to the distribution channel (2).

23. Installation (1, 1') according to one of Claims 1 to 21, **characterised in that** the connection channel is of telescopic construction.

24. Installation (1, 1') according to one of Claims 1 to 23, **characterised in that** an actuating device (49) is provided, on which a desired value for a variable that at least indirectly characterises a displacement distance s and/or a rotation angle can be preset, and which is connected to a transmission unit that converts the desired value into a set value.

25. Installation (1, 1') according to Claim 24, **characterised in that** the transmission unit comprises a gear that converts a rotary movement into a longitudinal movement.

26. Installation (1, 1') according to one of Claims 1 to 25, **characterised in that** the functional channel (5) is of telescopic construction.

27. Installation (1, 1') according to one of Claims 1 to 26, **characterised in that** the connection channel (3, 4) is formed by an element that forms a hollow body, which is connected to the distribution channel (2) and the connection channel through the opening area in a flow-carrying connection, and extends away from the distribution channel (2).

28. Installation (1, 1') according to one of Claims 1 to 27, **characterised in that** the functional channel (5) is formed by an element that forms a hollow body, the outer periphery of which has a surface containing holes, particularly a perforation, at least in parts.

29. Installation (1, 1') according to Claim 28, **characterised in that** the hollow body that forms the functional channel (5) consists at least partly of a perforated metal sheet with a support structure or a perforated metal foil.

30. Installation (1, 1') according to one of Claims 1 to 29, **characterised in that** the elements forming the individual channels (2, 3, 4, 5) are made of metal, plastics or inert material, preferably stainless steel.

31. Installation (1, 1') according to Claim 30, **characterised in that** the elements that form or carry the individual channels (2, 3, 4, 5) are made from stainless steel components which are joined together releasably or non-releasably.

32. Installation (1, 1') according to one of Claims 1 to 31, **characterised in that** the functional channel (5) is sectional and the surface zones (59, 60) that form the outlets (6.1, 6.n) are releasably connected to the base element that forms the functional channel (5).

33. Apparatus (44) for treating granular products by drying, film-coating or coating, especially for treating pharmaceutical particles
having a drum (61) mounted to be rotatable about an axis and adapted to be filled with the product (56);
having an installation (1, 1') mounted in the drum (61) for guiding a gas according to one of Claims 1 to 32.

34. Apparatus (44) according to Claim 33, **characterised in that** the functional channel (5) is arranged parallel to the rotation axis (R) of the drum (61).

35. Apparatus (44) according to Claim 33 or 34, **characterised in that** upstream of the installation (1, 1') is arranged an installation (57) for applying medium.

36. Use of an apparatus according to one of Claims 33 to 35 for producing dabigatran etexilate pellets or delayed-release dipyridamole forms.

## Revendications

1. Installation (1) pour guider un gaz destiné à des dispositifs (44) servant à traiter une matière granuleuse (56) par séchage, enrobage par un film ou enduction, notamment unité d'admission d'air, comportant un canal de distribution (2) central qui peut être relié à un dispositif d'approvisionnement en gaz et est relié à des sorties (6.1, 6.n) disposées à distance de celui-ci et disposées à distance dans le sens axial les unes par rapport aux autres pour le gaz via des canaux de liaison (3, 4), **caractérisée par** les caractéristiques suivantes :
- avec un canal fonctionnel (5) disposé à distance du canal de distribution (2) et présentant une pluralité de sorties (6.1, 6.n) ;
- le canal fonctionnel (5) comporte au moins deux zones d'admission (11, 12) disposées à distance axialement l'une par rapport à l'autre, chacune des zones d'admission (11, 12) étant reliée au canal de distribution (2) via un canal de liaison (3, 4) ;
- les sorties (6.1, 6.n) sont disposées, considérées dans le sens axial sur le canal fonctionnel (5), entre l'embouchure de deux canaux de liaison (3, 4) disposés l'un à côté de l'autre dans le sens axial dans le canal fonctionnel (5) et la position du canal fonctionnel (5) peut être modifiée par rapport au canal de distribution (2).

2. Installation (1') pour guider un gaz destiné à des dispositifs (44) servant à traiter une matière granuleuse (56) par séchage, enrobage par un film ou enduction, notamment unité d'admission d'air, comportant un canal de distribution (2) central qui peut être relié à un dispositif d'approvisionnement en gaz et est relié à des sorties (6.1, 6.n) disposées à distance de celui-ci et disposées à distance dans le sens axial les unes par rapport aux autres pour le gaz via des canaux de liaison (3, 4), **caractérisée par** les caractéristiques suivantes :
- avec un canal fonctionnel (5) disposé à distance du canal de distribution (2) et présentant une pluralité de sorties (6.1, 6.n) ;
- le canal fonctionnel (5) comporte une zone d'admission (11) qui est reliée au canal de distribution (2) via le canal de liaison (3), le canal de liaison (3) et le canal fonctionnel (5) étant disposés à un certain angle l'un par rapport à l'autre, et la position du canal fonctionnel (5) peut être modifiée par rapport au canal de distribution (2).

3. Installation (1') selon la revendication 2, **caractérisée en ce que** le canal fonctionnel (5) est logé en porte-à-faux sur le canal de liaison (3).

4. Installation (1, 1') selon l'une quelconque des revendications 1 à 3, **caractérisée en ce que** les sorties (6.1, 6.n) sont disposées au moins sur la zone dirigée vers le canal de distribution (2) de la surface extérieure (42) du canal fonctionnel (5).

5. Installation (1, 1') selon l'une quelconque des revendications 1 à 4, **caractérisée en ce que** les sorties (6.1, 6.n) sont disposées sur la zone dirigée loin du canal de distribution (2) de la surface extérieure (43) du canal fonctionnel (5).

6. Installation (1, 1') selon l'une quelconque des revendications 1 à 5, **caractérisée en ce que** les sorties (6.1, 6.n) recouvrent au moins une zone de surface (59, 60) prédéfinie sur la périphérie extérieure du canal fonctionnel (5).

7. Installation (1, 1') selon la revendication 6, **caractérisée en ce que** les sorties (6.1, 6.n) individuelles sont **caractérisées par** une section transversale de sortie avec une dimension comprise dans la plage de 0,1 à 1 mm, de préférence 0,2 à 0,6 mm, en particulier environ 0,5 mm et avec une distance prédéfinie comprise dans la plage de 0,5 à 2 mm, de préférence 0,8 à 1,5 mm, en particulier environ 1 mm les unes par rapport aux autres dans le sens axial et sont disposées perpendiculairement les unes par rapport aux autres.

8. Installation (1, 1') selon l'une quelconque des revendications 1 à 7, **caractérisée en ce que** les sorties (6.1, 6.n) sont disposées, considérées dans le sens axial, au moins sur une zone partielle du développement axial du canal fonctionnel (5).

9. Installation (1, 1') selon l'une quelconque des revendications 1 à 8, **caractérisée en ce que** le canal de distribution (2) et le canal fonctionnel (5) sont disposés parallèlement l'un à l'autre.

10. Installation (1, 1') selon l'une quelconque des revendications 1 à 9, **caractérisée en ce que** le canal de distribution (2) et le canal fonctionnel (5) sont disposés dans un angle l'un par rapport à l'autre.

11. Installation (1, 1') selon l'une quelconque des revendications 1 à 10, **caractérisée en ce que** le canal de distribution (2) et le canal de liaison (3, 4) individuel sont disposés perpendiculairement l'un par rapport à l'autre.

12. Installation (1, 1') selon l'une quelconque des revendications 1 à 11, **caractérisée en ce que** le canal fonctionnel (5) et le canal de liaison (3, 4) individuel sont disposés perpendiculairement l'un par rapport à l'autre.

13. Installation (1, 1') selon l'une quelconque des revendications 1 à 12, **caractérisée en ce que** le canal de distribution (2), le canal fonctionnel (5) et le canal de liaison (3, 4) individuel sont **caractérisés** chacun par une géométrie de section transversale quelconque.

14. Installation (1, 1') selon l'une quelconque des revendications 1 à 13, **caractérisée en ce que** le canal fonctionnel (5) est réalisé, considéré en section transversale, comme une aile et est formé par un côté supérieur (42) et un côté inférieur (43) reliés l'un à l'autre par des transitions arrondies, le côté supérieur et le côté inférieur (42, 43) étant **caractérisés**, considérés en section transversale, chacun par la juxtaposition de zones de surface (42.1, 42.2, 43.1, 43.2) orientées et configurées différemment.

15. Installation (1, 1') selon la revendication 14, **caractérisée en ce que** le canal fonctionnel (5) est réalisé de manière symétrique en section transversale par rapport à un plan.

16. Installation (1, 1') selon l'une quelconque des revendications 1 à 15, **caractérisée en ce que** le canal fonctionnel (5) peut être pivoté autour de son axe longitudinal (A5).

17. Installation (1, 1') selon l'une quelconque des revendications 1 à 16, **caractérisée en ce que** le canal fonctionnel (5) peut être déplacé parallèlement au canal de liaison (3) individuel ou canaux de liaison (3, 4).

18. Installation (1, 1') selon l'une quelconque des revendications 1 à 17, **caractérisée en ce qu'**au moins un dispositif de réglage (45) pour la modification de la position est associé au canal fonctionnel (5), le dispositif de réglage fonctionnant selon l'un des principes actifs suivants :
- mécanique
- hydraulique
- pneumatique
- électronique
- une combinaison des principes précités.

19. Installation (1, 1') selon la revendication 18, **caractérisée en ce que** le dispositif de réglage (45) est intégré dans le canal de distribution (2) et le ou les canaux de liaison (3, 4).

20. Installation (1, 1') selon la revendication 18, **caractérisé en ce que** le dispositif de réglage (45) est disposé en dehors du canal de distribution (2) et du canal de liaison (3) ou des canaux de liaison (3, 4).

21. Installation (1, 1') selon l'une quelconque des revendications 1 à 20, **caractérisée en ce que** le canal de liaison (3, 4) individuel ne peut pas être modifié dans sa longueur.

22. Installation (1, 1') selon l'une quelconque des revendications 1 à 21, **caractérisée en ce que** le canal fonctionnel (5) est logé, dans sa zone d'admission ou zones d'admission (11, 12), respectivement dans un coulisseau (46.1, 46.2) qui peut être guidé sur les éléments (23, 24) formant les canaux de liaison (3, 4) dans un guidage (47.1, 47.2), dans chaque position du coulisseau (46.1, 46.2) par rapport au guidage (47.1, 47.2), le canal fonctionnel (5) étant relié de manière à guider l'écoulement au canal de distribution (2).

23. Installation (1, 1') selon l'une quelconque des revendications 1 à 21, **caractérisée en ce que** le canal de liaison est réalisé de manière télescopique.

24. Installation (1, 1') selon l'une quelconque des revendications 1 à 23, **caractérisée en ce qu'**un dispositif d'actionnement (49) est prévu, au niveau duquel une valeur de consigne peut être prescrite pour une grandeur caractérisant au moins indirectement une course de réglage s et/ou un angle de rotation et qui est relié à une unité de transfert qui convertit la grandeur de consigne en une grandeur de réglage.

25. Installation (1, 1') selon la revendication 24, **caractérisée en ce que** l'unité de transfert comporte un engrenage qui convertit un mouvement de rotation en un mouvement longitudinal.

26. Installation (1, 1') selon l'une quelconque des revendications 1 à 25, **caractérisée en ce que** le canal fonctionnel (5) est réalisé de manière télescopique.

27. Installation (1, 1') selon l'une quelconque des revendications 1 à 26, **caractérisée en ce que** le canal de liaison (3, 4) est formé par un élément formant un corps creux qui se trouve en liaison de guidage d'écoulement respectivement avec le canal de distribution (2) et le canal de liaison par la zone d'ouverture, et s'étend loin du canal de distribution (2).

28. Installation (1, 1') selon l'une quelconque des revendications 1 à 27, **caractérisée en ce que** le canal fonctionnel (5) est formé par un élément formant un corps creux, dont la périphérie extérieure présente au moins dans des zones partielles une surface trouée, en particulier une perforation.

29. Installation (1, 1') selon la revendication 28, **caractérisée en ce que** le corps creux formant le canal fonctionnel (5) se compose au moins partiellement d'une tôle perforée avec une construction d'appui ou d'une feuille métallique perforée.

30. Installation (1, 1') selon l'une quelconque des revendications 1 à 29, **caractérisée en ce que** les éléments formant les canaux individuels (2, 3, 4, 5) sont en métal, en plastique ou en un matériau inerte, de préférence en acier spécial.

31. Installation (1, 1') selon la revendication 30, **caractérisée en ce que** les éléments portant ou formant les canaux individuels (2, 3, 4, 5) sont formés par des composants en acier spécial qui sont reliés chacun de manière amovible ou inamovible les uns aux autres.

32. Installation (1, 1') selon l'une quelconque des revendications 1 à 31, **caractérisée en ce que** le canal fonctionnel (5) est réalisé en plusieurs parties et les zones de surface (59, 60) formant les sorties (6.1, 6.n) sont reliées de manière amovible à l'élément de base formant le canal fonctionnel (5).

33. Dispositif (44) servant à traiter une matière granuleuse par séchage, enrobage par un film ou enduction, en particulier pour le traitement de particules pharmaceutiques
avec un tambour (61) logé de manière rotative autour d'un axe et pouvant être rempli avec la matière (56) ;
avec une installation (1, 1') disposée dans le tambour (61) pour guider un gaz selon l'une quelconque des revendications 1 à 32.

34. Dispositif (44) selon la revendication 33, **caractérisé en ce que** le canal fonctionnel (5) est disposé parallèlement à l'axe de rotation (R) du tambour (61).

35. Dispositif (44) selon la revendication 33 ou 34, **caractérisé en ce qu'**en amont du dispositif (1, 1') est disposée une installation (57) pour l'application de produit.

36. Utilisation d'un dispositif selon l'une quelconque des revendications 33 à 35 pour la fabrication de pastilles de dabigatran etexilate ou de formes retard de dipyridamole.
